(19) Europäisches Patentamt
European Patent Office
Office européen des brevets

(11) EP 4 112 040 A1

(12) EUROPEAN PATENT APPLICATION
published in accordance with Art. 153(4) EPC

(43) Date of publication:
**04.01.2023 Bulletin 2023/01**

(21) Application number: **21761787.7**

(22) Date of filing: **25.02.2021**

(51) International Patent Classification (IPC):
***A61K 8/89*** (2006.01) ***A61Q 19/00*** (2006.01)

(52) Cooperative Patent Classification (CPC):
**A61K 8/89; A61Q 19/00**

(86) International application number:
**PCT/JP2021/007182**

(87) International publication number:
**WO 2021/172463 (02.09.2021 Gazette 2021/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.02.2020 JP 2020029779**

(71) Applicant: **Shiseido Company, Ltd.**
**Chuo-ku**
**Tokyo 104-0061 (JP)**

(72) Inventors:
- **MATSUKURA, Toshihiko**
  **Tokyo 104-0061 (JP)**
- **OGUCHI, Nozomi**
  **Tokyo 104-0061 (JP)**

(74) Representative: **Cabinet Beau de Loménie**
**158, rue de l'Université**
**75340 Paris Cedex 07 (FR)**

# (54) COSMETIC CONTAINING SILOXANE LINKAGE-CONTAINING POLYMER COMPOUND HAVING HOST GROUP AND/OR GUEST GROUP

(57) An object of the present disclosure is to provide a cosmetic material having excellent feeling of use. The present invention relates to a cosmetic material comprising a host body consisting of a polymer compound having a host group and a guest body consisting of a polymer compound having a guest group, or a host-guest body consisting of a polymer compound having a host group and a guest group, wherein, at least one of the polymer compound having the host group or the polymer compound having the guest group, or the polymer compound having the host group and the guest group has a siloxane bond.



Processed by Luminess, 75001 PARIS (FR)

## Description

FIELD

The present disclosure relates to a cosmetic material comprising a polymer compound having a siloxane bond and a host group and/or guest group.

BACKGROUND

It is known to form a film by using a cross-linked polymer compound. In particular, a film-forming agent containing a cross-linked silicone resin has been widely used as various coating agents and cosmetics.
Patent Document 1 discloses a cosmetic material containing trimethylsiloxysilicate, etc., as a film-forming agent.
Further, it is known that a host body consisting of a polymer compound having a host group and a guest body consisting of a polymer compound having a guest group, or a host-guest body consisting of a polymer compound having a host group and guest group can reversibly form a bond through a combination of the host group and the guest group (Patent Document 2).
Patent Document 3 discloses a method for producing an aqueous solution of a monomer having a host group, a monomer having a guest group, and an acrylic monomer.

[CITATION LIST]

[PATENT LITERATURE]

[Patent Document 1] WO2019-99576A
[Patent Document 2] WO2012/036069A
[Patent Document 3] WO2013/162019A

SUMMARY

[TECHNICAL PROBLEM]

Conventional cosmetic materials containing a cross-linked polymer compound have sometimes insufficient feeling of use. Specifically, in a conventional cosmetic material containing a cross-linked polymer compound, after application to skin, etc., it has sometimes an undesirable feeling of use in terms of feeling of close adhesion, feeling of resilience, and/or feeling of tightness.
Accordingly, it is an object of the present disclosure to provide a cosmetic material having excellent feeling of use.
The present inventors have intensively studied to solve such problems, and have conceived of the present invention.

[SOLUTION TO PROBLEM]

The invention according to the present disclosure includes the following embodiments:

<Embodiment 1>

A cosmetic material comprising a host body consisting of a polymer compound having a host group and a guest body consisting of a polymer compound having a guest group, or a host-guest body consisting of a polymer compound having a host group and a guest group, wherein, at least one of the polymer compound having the host group and the polymer compound having the guest group, or the polymer compound having the host group and the guest group, has a siloxane bond.

<Embodiment 2>

The cosmetic material according to embodiment 1, wherein the polymer compound having the host group and the polymer compound having the guest group have a siloxane bond.

<Embodiment 3>

The cosmetic material according to embodiment 1, wherein at least one of the polymer compound having the host group and the polymer compound having the guest group, or the polymer compound having the host group and the guest group, has a siloxane bond in the main chain of the polymer.

<Embodiment 4>

The cosmetic material according to embodiment 3, wherein the polymer compound having the host group and the polymer compound having the guest group have a siloxane bond in the main chain of the polymer.

<Embodiment 5>

The cosmetic material according to one of embodiments 1 to 4, wherein

the polymer compound having the host group has the host group in a side chain thereof, and
the polymer compound having the guest group has the guest group in a side chain thereof.

<Embodiment 6>

The cosmetic material according to one of embodiments 1 to 4, wherein

the polymer compound having the host group has the host group in a side chain thereof, and
the polymer compound having the guest group has the guest group in a terminal thereof.

<Embodiment 7>

The cosmetic material according to one of embodiments 1 to 4, wherein

the polymer compound having the host group has the host group in a terminal thereof, and
the polymer compound having the guest group has the guest group in a side chain thereof.

<Embodiment 8>

The cosmetic material according to one of embodiments 1 to 7, wherein at least one of the polymer compound having the host group and the polymer compound having the guest group, or the polymer compound having the host group and the guest group, is cross-linked.

<Embodiment 9>

The cosmetic material according to one of embodiments 1 to 8, wherein at least one of the polymer compound having the host group and the polymer compound having the guest group, or the polymer compound having the host group and the guest group, has a structure represented by the following formula (1):

[Chem 1]

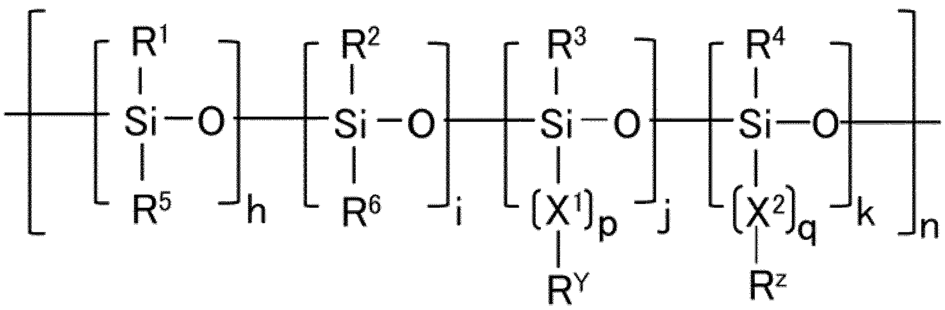

・・・(1)

[In Formula (1), $R^1$ to $R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, a monovalent spirocyclic compound, a monovalent condensed-ring compound, a group represented by $-R^{R1}-COOH$, or a group represented by $-R^{R2}-C(O)O-R^{R3}$, wherein these groups may have a substituent, and wherein $R^{R1}$, $R^{R2}$ and $R^{R3}$ are each an alkyl group or alkylene group having 1 to 10 carbon atoms; $X^1$ and $X^2$ are each independently O, $Si(OH)_2$, $Si(R^{10})_2$, N(H) or $N(COCH_3)$, or an urethane bond, an urea bond, an ether bond, an amide bond, or an ester bond, or a carbonyl group, an alkylene group, a cycloalkylene group, an alkenylene group, an alkoxylene group, a divalent heterocyclic group, an urethane group, an urea group, or an arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j, and k each represent an integer of 0 or more; and they may be the same or different from each other; at least j or k represents an integer of 1 or more; n represents an integer of 1 or more.]

<Embodiment 10>

The cosmetic material according to embodiment 9, wherein the polymer compound having the host group and the polymer compound having the guest group have the structure represented by the formula (1).

<Embodiment 11>

The cosmetic material according to embodiment 9 or 10, wherein at least one of the polymer compound having the host group and the polymer compound having the guest group, or the polymer compound having the host group and the guest group, has the structure represented by the formula (1) in the main chain of the polymer.

<Embodiment 12>

The cosmetic material according to embodiment 9 or 10, wherein at least one of the polymer compound having the host group and the polymer compound having the guest group, or the polymer compound having the host group and the guest group, has:

a vinyl main chain, an acryl main chain, an urethane main chain, an epoxy main chain, a polyimide main chain, a polyester main chain, a poly-urea main chain, or a polycarbonate main chain, and
a side chain having the structure represented by the formula (1).

<Embodiment 13>

The cosmetic material according to one of embodiments 1 to 12, wherein at least one of the polymer compound having the host group and the polymer compound having the guest group, or the polymer compound having the host group and the guest group, has a structural unit represented by the following formula (2):

[Chem 2]

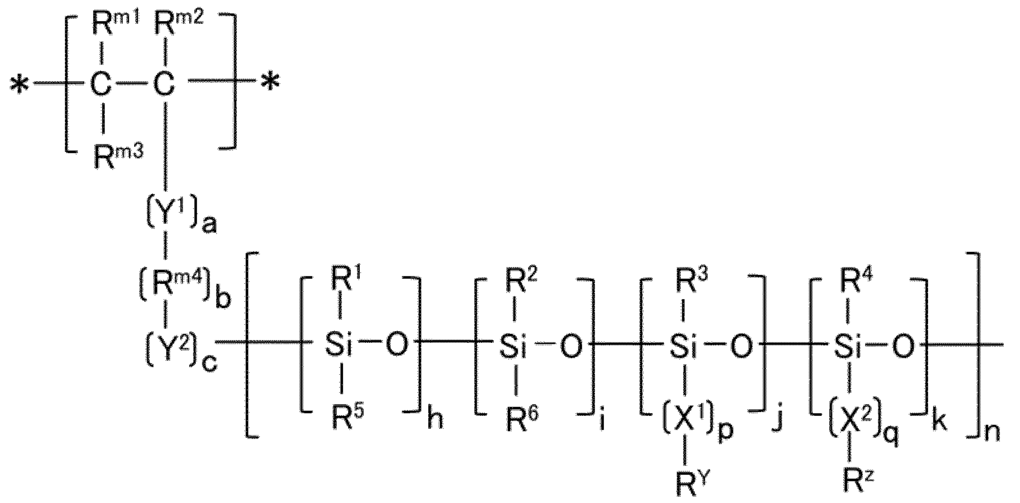

[In Formula (2), $R^1$-$R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, a monovalent spirocyclic compound, a monovalent condensed-ring compound, a group represented by $-R^{R1}$-COOH, or a group represented by $-R^{R2}$-C(O)O-$R^{R3}$, wherein these groups may have a substituent, and wherein $R^{R1}$, $R^{R2}$ and $R^{R3}$ are each an alkyl group or alkylene group having 1 to 10 carbon atoms; $X^1$ and $X^2$ are each independently O, $Si(OH)_2$, $Si(R^{10})_2$, N(H), or $N(COCH_3)$, or an urethane bond, an urea bond, an ether bond, an amide bond, or an ester bond, or a carbonyl group, an alkylene group, a cycloalkylene group, an alkenylene group, an alkoxylene group, a divalent heterocyclic group, an urethane group, an urea group, or an arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j, and k each represent an integer of 0 or more, these may be the same or different from each other, and at least j or k is an integer of 1 or more; n represents an integer of 1 or more; $R^{m1}$ to $R^{m3}$ are each independently a hydrogen atom or an alkyl group, $Y^1$ is an ether bond, an amide bond, or an ester bond; $R^{m4}$ is an alkylene group, a cycloalkylene group, an alkenylene group, an alkoxylene group, or an arylene group, and these groups may have a substituent, and $Y^2$ is an ether bond, an amide bond, or an ester bond, O, Si $(OH)_2$, $Si(R^{10})_2$, N(H), or $N(COCH_3)$; and a, b, and c each independently represent an integer of 0 to 3; * represents a single bond constituting the main chain of the polymer compound.]

<Embodiment 14>

The cosmetic material according to embodiment 13, wherein the polymer compound having the host group and the polymer compound having the guest group have the structure represented by the formula (2).

<Embodiment 15>

The cosmetic material according to embodiment 13 or 14, wherein at least one of the polymer compound having the host group and the polymer compound having the guest group, or the polymer compound having the host group and the guest group, further has a structural unit represented by the following formula (3):

[Chem 3]

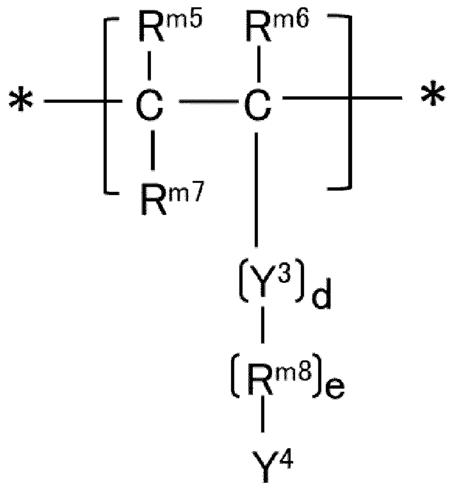

...(3)

[In Formula (3), $R^{m5}$ to $R^{m7}$ are each independently a hydrogen atom or an alkyl group; $Y^3$ is an ether bond, an amide bond, or an ester bond; $R^{m8}$ is an alkylene group, a cycloalkylene group, an alkenylene bond, an alkoxylene group, or an arylene group, and these groups may have a substituent; $Y^4$ is a hydrogen atom, or an alkyl group, a hydroxyl group, $Si(OH)_3$, $Si(R^{10})_3$, $NH_2$, $C(O)CH_3$, $C(O)NH_2$, or $N(COCH_3)$); d and e are each independently an integer of 0 to 3; * represents a single bond constituting the main chain of the polymer compound.]

<Embodiment 16>

The cosmetic material according to one of embodiments 9 to 15, wherein, in the formula (1) and/or formula (2),

the host group represented by $R^Y$ is $\alpha$-cyclodextrin, $\beta$- cyclodextrin or $\gamma$- cyclodextrin, and
the guest group represented by $R^Z$ is an alkyl group which can have a substituent, or an aryl group which can have a substituent.

<Embodiment 17>

The cosmetic material according to one of embodiments 9 to 16, wherein, in the formula (1) and/or formula (2), $R^1$, $R^2$, $R^3$, $R^4$ and $R^5$ are a methyl group.

<Embodiment 18>

The cosmetic material according to one of embodiments 9 to 16, wherein, in the formula (1) and/or formula (2),

k=0, and
$R^1$, $R^2$, $R^3$, $R^5$ and $R^6$ are an alkyl group.

<Embodiment 19>

The cosmetic material according to one of embodiments 9 to 16, wherein, in the formula (1) and/or formula (2),

k=0
$R^1$, $R^2$, $R^3$ and $R^5$ are a methyl group, and
$R^6$ is a pentyl group.

<Embodiment 20>

The cosmetic material according to one of embodiments 9 to 19, wherein, in the formula (1) and/or formula (2),

$(X^1)_p$ is $-(CH_2)_3-N(COCH_3)-$ and j is an integer of 1 or more,

and/or
$(X^2)_q$ is $-R^{11}$-CO-O- or $-R^{11}$-CO-NH-, wherein $R^{11}$ is an alkylene group having 1 to 12 carbon atoms, which can have a substituent, and k is an integer of 1 or more.

<Embodiment 21>

The cosmetic material according to one of embodiments 1 to 20, wherein the host group and the guest group are selected from one of the following combinations (a) to (c):

(a) the host group is α-cyclodextrin and
the guest group is at least one selected from the group consisting of (1) a linear alkyl group of 4 to 18 carbon atoms, (2) a linear alkyl group of 4 to 18 carbon atoms having a hydroxy group, (3) a linear alkyl group of 4 to 18 carbon atoms having a carboxyl group, (4) a linear alkyl group of 4 to 18 carbon atoms having an amino group, (5) a cyclic alkyl group, (6) a phenyl group, (7) an azobenzene group, and (8) a cinnamic acid group;
(b) the host group is β-cyclodextrin and
the guest group is at least one selected from the group consisting of (1') t-butyl group, (2') an adamantyl group, (3') an aryl group, (4') an aryl group having a hydroxyl group, (5') an aryl group having a carboxyl group, (6') an aryl group having an amino group, (7') a ferrocenyl group, (8') an azobenzene group, and (9') a dansyl group;
(c) the host group is γ-cyclodextrin and

the guest group is at least one selected from the group consisting of (1") an alkyl group of up to 18 carbon atoms, (2") an alkyl group of up to 18 carbon atoms having a hydroxy group, (3") an alkyl group of up to 18 carbon atoms having a carboxyl group, (4") an alkyl group of up to 18 carbon atoms having an amino group, (5") an adamantyl group, (6") a group having clusters composed of carbon atoms, (7") a dansyl group having an aryl group, (8") a ferrocenyl group, and (9") an anthracenyl group.

<Embodiment 22>

The cosmetic material according to one of embodiments 1 to 19, further comprising a solvent.

<Embodiment 23>

The cosmetic material according to embodiment 20, wherein the solvent is volatile silicone or a volatile hydrocarbon compound.

[ADVANTAGEOUS EFFECTS OF INVENTION]

According to the present disclosure, there is provided a cosmetic material having excellent feeling of use.

DESCRIPTION OF EMBODIMENTS

<<Cosmetic material>>

The cosmetic material according to the present disclosure:

comprises a host body consisting of a polymer compound having a host group and a guest body consisting of a polymer compound having a guest group, or a host-guest body consisting of a polymer compound having a host group and a guest group,
wherein, at least one of the polymer compound having the host group and the polymer compound having the guest group, or the polymer compound having the host group and the guest group, has a siloxane bond.

In a conventional cosmetic material, in which a film is formed by cross-linking a polymer compound via a covalent bond, there has been a case where a good feeling of use cannot be obtained when applied to skin, etc.
On the other hand, the inventors of the present application have found that a cosmetic material containing a polymer compound having a host group and/or guest group exhibits excellent feeling of use as compared with the conventional cosmetic material. Specifically, it has been found that a cosmetic material containing a polymer compound having a siloxane bond and a host group and/or guest group exhibits excellent characteristics in terms of feeling of close adhesion, feeling of resilience, and feeling of tightness. Further, the cosmetic material according to the present disclosure

also exhibits excellent durability of cosmetic material.

Without wishing to be bound by theory, it is considered that the cosmetic material according to the present disclosure contains a polymer compound having a host group and/or guest group, and therefore, when applied to the skin, etc., the polymer compounds are bonded to each other via a host-guest interaction to form a film (a cosmetic coating film). Therefore, it is considered that the cosmetic material according to the present disclosure exhibits excellent characteristics in the feeling of resilience.

Further, without wishing to be bound by theory, since the bonding via the host-guest interaction is based on non-covalent bonding, it is considered that the cosmetic coating film formed by the cosmetic material according to the present disclosure is more elastic (smaller Young's modulus) than the cosmetic coating film formed by a covalent bonding between polymers, and as a result, exhibits excellent characteristics with respect to the feeling of close adhesion and the feeling of tightness.

Further, since the cosmetic material according to the present disclosure uses a polymer compound having a siloxane bond, it is possible to provide a cosmetic material which is chemically particularly stable.

Further, a cosmetic material containing a polymer compound having a siloxane bond has relatively high solubility in a nonpolar solvent such as hydrocarbon oil or silicone oil. The solubility of the conventional polymer compound having a host group and/or guest group in a solvent is sometimes insufficient. On the other hand, the polymer compound contained in the cosmetic material according to the present disclosure has a siloxane bond, thereby the solubility is improved. When the solubility in a volatile nonpolar solvent is relatively high, it becomes easy to apply a cosmetic material to the skin, etc., by coating, and it becomes possible to form a uniform and thin film relatively easily.

The cosmetic material according to the present disclosure can be suitably used, in particular, as an ultraviolet protective cosmetic, such as a sunscreen milk and a sunscreen cream; and as a makeup cosmetic, such as a cosmetic base (base makeup), a foundation, a concealer, a blusher, an eyeshadow, a mascara, an eyeliner, an eyebrow, an overcoat agent, and a lipstick.

<Host group and guest group>

"Host-guest interaction" refers to a bond formed between a host group and a guest group. The host group binds to the guest group through the inclusion of the guest group. The host-guest interaction occurs when the size of the guest group is suitable to be incorporated into the interior space of the host group and when the interaction between the host group and the guest group includes at least one of a hydrophobic interaction, a hydrogen bonding, an electrostatic interaction, and a coordination bonding.

(Host group)

Examples of the host group include cyclodextrin (CD). Specific examples thereof include α-cyclodextrin, β-cyclodextrin, and γ-cyclodextrin. When these groups are used as a host group, a stable host-guest interaction can be formed.

(Guest group)

The guest group is not particularly limited as long as it can act as a guest group with respect to the corresponding host group. Examples of the guest group include an alkyl group which may have a substituent, and an aryl group which may have a substituent. As the guest group, the alkyl group and aryl group which may have a substituent have 1 to 18 carbon atoms, preferably 3 to 12 carbon atoms, and more preferably 3 to 9 carbon atoms. Examples of the guest group further include a trialkylsilyl group (e.g., a trimethylsilyl group, a triethylsilyl group, and a tripropylsilyl group, particularly a trimethylsilyl group).

Among the guest group, examples of the alkyl group which may have a substituent include a linear, branched or cyclic alkyl group with 1 to 18 carbon atoms (C1 to C18). Specific examples thereof include an alkyl group such as methyl, ethyl, n-propyl, isopropyl, n-butyl, isobutyl, sec-butyl, tert-butyl, pentyl, cyclopentyl, hexyl, cyclohexyl, isohexyl, dodecyl, octadecyl, and adamantyl group. Of these, the adamantyl group or the butyl group are preferable, and the adamantyl group is particularly preferable. The alkyl group may have, for example, 1 to 3 substituents, such as a halogen atom (e.g., fluorine, chlorine, bromine, etc.), a carboxyl group, an ester group, an amide group, or a hydroxyl group which may be optionally protected. It may also be an alkyl group obtained by bonding ferrocene, which is an organometallic complex, as a substituent.

Among the guest group, examples of the aryl group which may have a substituent include an aryl group having a single ring or two or more rings. Specific examples thereof include phenyl, triyl, xylyl, naphthyl, anthryl, and phenanthryl group. The aryl group may have, for example, 1 to 3 substituent, such as an alkyl group (e.g., C1 to C18 alkyl groups), a halogen atom (e.g., fluorine, chlorine, bromine, etc.), a carboxyl group, an ester group, an amide group, an azo group

with an aryl group, or a hydroxyl group which may be protected.

(Combinations of host group and guest group)

In one embodiment according to the present disclosure, the host group and the guest group in the cosmetic material are any one of the following combinations (a) to (c):

(a) the host group is an α-cyclodextrin and
the guest group is at least one selected from the group consisting of (1) a linear alkyl group of 4 to 18 carbon atoms, (2) a linear alkyl group of 4 to 18 carbon atoms having a hydroxy group, (3) a linear alkyl group of 4 to 18 carbon atoms having a carboxyl group, (4) a linear alkyl group of 4 to 18 carbon atoms having an amino group, (5) a cyclic alkyl group, (6) a phenyl group, (7) an azobenzene group, and (8) a cinnamic acid group;
(b) the host group is β-cyclodextrin and
the guest group is at least one selected from the group consisting of (1') t-butyl group, (2') an adamantyl group, (3') an aryl group, (4') an aryl group having a hydroxyl group, (5') an aryl group having a carboxyl group, (6') an aryl group having an amino group, (7') a ferrocenyl group, (8') an azobenzene group, and (9') a dansyl group;
(c) the host group is γ-cyclodextrin and

the guest group is at least one selected from the group consisting of (1") an alkyl group of up to 18 carbon atoms, (2") an alkyl group of up to 18 carbon atoms having a hydroxy group, (3") an alkyl group of up to 18 carbon atoms having a carboxyl group, (4") an alkyl group of up to 18 carbon atoms having an amino group, (5") an adamantyl group, (6") a group having clusters composed of carbon atoms, (7") a dansyl group having an aryl group, (8") a ferrocenyl group, and (9") an anthracenyl group.

<Polymer compound>

The term "polymer compound" means a polymer formed from one or more kinds of monomers, and in particular, a polymer having a molecular weight distribution and having a number average molecular weight in terms of polystyrene of $1\times10^3$ or more (e.g., $1\times10^3$ to $1\times10^8$). The polymer compound may be a block copolymer, a random copolymer, an alternating copolymer, or a graft copolymer, or may be another type of polymer.

The polymer compound having the host group and/or guest group according to the present disclosure may be a polymer formed from a single monomer, or may be a copolymer, a block copolymer, or a graft copolymer.

When the polymer compound having a host group and/or guest group according to the present disclosure contains a siloxane bond, the silicon atom (Si) constituting the siloxane bond preferably has a hydrogen atom or a group represented by $R^S$. Here, $R^S$ is an alkyl group, a cycloalkyl group, an alkenyl group, an alkoxy group, a cycloalkoxy group, a hydroxy group, a carboxyl group, an aldehyde group, an aryl group, an aryloxy group, or a monovalent heterocyclic group, and these groups may have a substituent; if there are multiple $R^S$, they may be identical or different from each other. In particular, $R^S$ may be an alkyl group having 1 to 12 carbon atoms or an aryl group which may have a substituent. $R^S$ is preferably an alkyl group having 1 to 12 carbon atoms, more preferably 1 to 6 carbon atoms, and particularly preferably 1 or 2 carbon atoms.

Preferably, at least one of the polymer compound having a host group or the polymer compound having a guest group, or the polymer compound having a host group and a guest group, has a polyorganosiloxane main chain. When the polymer compound contained in the cosmetic material according to the present disclosure has a siloxane bond in the main chain thereof, or, has a polyorganosiloxane main chain, it is preferable because chemical stability of the cosmetic material is further improved, and because relatively excellent solubility in hydrocarbon oil and silicone oil is obtained.

The polymer compound according to the present disclosure may have a polyorganosiloxane unit in the main chain or a side chain thereof. When the polymer compound according to the present disclosure contains a polyorganosiloxane unit, the proportion of the polyorganosiloxane unit may be 20% by weight or more, 30% by weight or more, or 40% by weight or more, and/or may be 100% by weight or less, 90% by weight or less, 80% by weight or less, 70% by weight or less, or 60% by weight or less, based on the entire polymer compound. When the proportion of the polyorganosiloxane unit is within this range, the chemical stability and solubility of the polymer compound are further improved.

Particularly preferably, both of the polymer compound having a host group and the polymer compound having a guest group contain a siloxane bond in the main chain of the polymer. When the polymer compound having a host group and the polymer compound having a guest group contained in the cosmetic material according to the present disclosure both contain a siloxane bond in the main chain thereof, the solubility in hydrocarbon oi and silicone oil are further improved.

When the polymer compound having a host group and/or guest group according to the present disclosure contains a siloxane bond, examples of such a polymer compound (hereinafter also referred to as a "siloxane polymer

compound") include polyorganosiloxane; a block copolymer of a polyorganosiloxane and a vinyl resin, an acrylic resin, an urethane resin, an epoxy resin, a polyimide resin, a polyester resin, or a polycarbonate resin; and a graft copolymer in which a polyorganosiloxane is grafted to a vinyl resin, an acrylic resin, an urethane resin, an epoxy resin, a polyimide resin, a polyester resin, or a polycarbonate resin. These resins and/or polyorganosiloxanes may have a substituent other than the host group and/or guest group. Further, when the polymer compound having a host group and/or guest group according to the present disclosure contains a siloxane bond, examples of such a polymer compound include an acrylic silicone resin, a graft copolymer based on acrylic silicone, a copolymer of polynorbornene and silicone, and a copolymer of pullulan and silicone.

Examples of polyorganosiloxane include methylpolysiloxane, phenylpolysiloxane, and methylphenylpolysiloxane, and these polysiloxanes may have a substituent other than the host group and/or guest group. The polymer compound according to the present disclosure particularly preferably has a methylpolysiloxane skeleton.

Preferably, at least one of the polymer compound having a host group and the polymer compound having a guest group, or the polymer compound having a host group and a guest group, has a structure represented by the following formula (1):

[Chem 4]

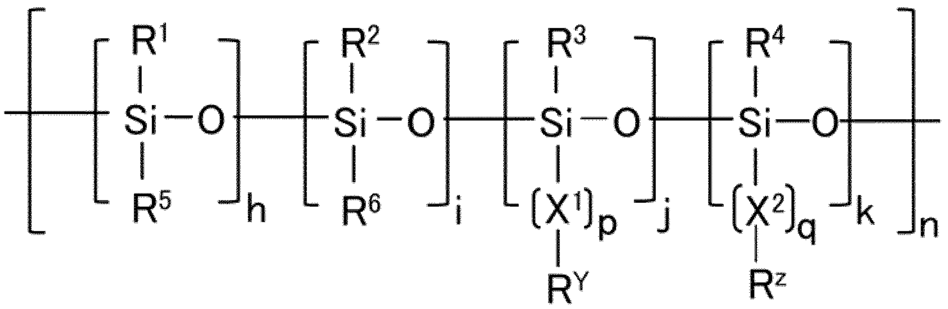

···(1)

[In Formula (1), $R^1$ to $R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, a monovalent spirocyclic compound, a monovalent condensed-ring compound, a group represented by $-R^{R1}-COOH$, or a group represented by $-R^{R2}-C(O)O-R^{R3}$, wherein these groups may have a substituent, and wherein $R^{R1}$, $R^{R2}$ and $R^{R3}$ are each an alkyl group or an alkylene group having 1 to 10 carbon atoms; $X^1$ and $X^2$ are each independently O, $Si(OH)_2$, $Si(R^{10})_2$, N(H) or $N(COCH_3)$, or an urethane bond, an urea bond, an ether bond, amide bond or an ester bond, or a carbonyl group, an alkylene group, a cycloalkylene group, an alkenylene group, an alkoxylene group, a divalent heterocyclic group, an urethane group, an urea group, or an arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j and k each represent an integer of 0 or more; and they may be the same or different from each other; at least j or k represents an integer of 1 or more; n represents an integer of 1 or more.]

Preferably, in the cosmetic material according to the present disclosure, the polymer compound having a host group and the polymer compound having a guest group have the structure represented by the above formula (1).

When the polymer compound contained in the cosmetic material according to the present disclosure has the structure represented by the above formula (1), it is possible that the polymer compound has a structure in which a terminal group is bonded to both ends of the structure represented by the above formula (1).

As the terminal group ($R^E$) which can be bonded to the terminal of the structure represented by the above formula (1), mention may be made of an alkyl group having 1 to 10 carbon atoms, in particular an alkyl group having 1 to 6 carbon atoms, or a group represented by $Si(R^{E1})_3$, wherein $R^{E1}$ is a hydrogen atom or an alkyl group having 1 to 10 carbon atoms, or an aryl group.

In a preferred embodiment according to the present disclosure, at least one of the polymer compound having a host group and the polymer compound having a guest group, or the polymer compound having a host group and a guest group, has the structure represented by the above formula (1) in the main chain of the polymer.

The polymer compound according to the present disclosure may be, for example, a block copolymer of a vinyl resin, an acrylic resin, a urethane resin, an epoxy resin, a polyimide resin, a polyester resin, or a polycarbonate resin,

and a polyorganosiloxane having the structure represented by the above formula (1).

In another preferred embodiment according to the present disclosure, the polymer compound contained in the cosmetic material has:

a vinyl backbone (i.e., a vinyl main chain), an acrylic backbone, an urethane backbone, an epoxy backbone, a polyimide backbone, a polyester backbone, a polyurea backbone, or a polycarbonate backbone, and
a side chain having the structure represented by the above formula (1).

Specifically, the polymer compound according to the present disclosure may be, for example, a graft polymer in which the polyorganosiloxane having the structure represented by the above formula (1) is grafted to a vinyl resin, an acrylic resin, an urethane resin, an epoxy resin, a polyurea resin, a polyimide resin, a polyester resin, or a polycarbonate resin.

In another preferred embodiment according to the present disclosure, at least one of the polymer compound having a host group and the polymer compound having a guest group, or the polymer compound having a host group and a guest group, has the structural unit represented by the following formula (2):

[Chem 5]

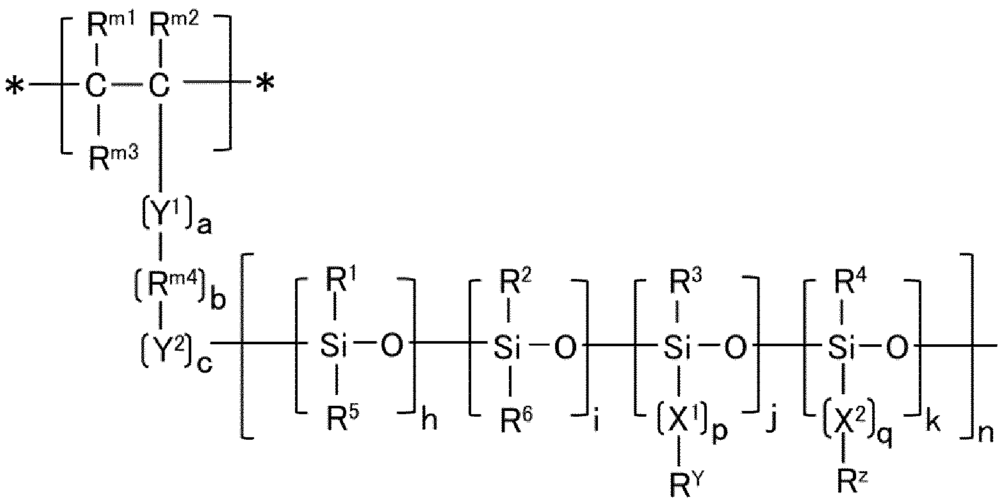

···(2)

[In Formula (2), $R^1$-$R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, an urethane group, an urea group, a heterocyclic group, a monovalent spirocyclic compound, a monovalent condensed-ring compound, a group represented by $-R^{R1}$-COOH, or a group represented by $-R^{R2}$-C(O)O-$R^{R3}$, wherein these groups may have a substituent, and wherein $R^{R1}$, $R^{R2}$ and $R^{R3}$ are each an alkyl group or alkylene group having 1 to 10 carbon atoms; $X^1$ and $X^2$ are each independently O, $Si(OH)_2$, $Si(R^{10})_2$, N(H), or $N(COCH_3)$, or an urethane bond, an urea bond, or an ether bond, an amide bond, or an ester bond, or a carbonyl group, an alkylene group, a cycloalkylene group, an alkenylene group, an alkoxylene group, a divalent heterocyclic group, an urethane group, an urea group, or arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j and k each represent an integer of 0 or more, these may be the same or different from each other, and at least j or k is an integer of 1 or more; n represents an integer of 1 or more; $R^{m1}$ to $R^{m3}$ are each independently a hydrogen atom or an alkyl group, $Y^1$ is an ether bond, an amide bond, or an ester bond; $R^{m4}$ is an alkylene group, a cycloalkylene group, an alkenylene group, an alkoxylene group, or an arylene group, and these groups may have a substituent, and $Y^2$ is an ether bond, an amide bond, or an ester bond, O, $Si(OH)_2$, $Si(R^{10})_2$, N(H), or $N(COCH_3)$; and a, b and c each independently represent an integer of 0 to 3; * represents a single bond constituting the main chain of the polymer compound.]

In a preferred embodiment according to the present disclosure, at least one of the polymer compound having a host group or the polymer compound having a guest group, or the polymer compound having a host group and a guest group, further has, in addition to the structural unit represented by the above formula (2), a structural unit represented

by the following formula (3):

[Chem 6]

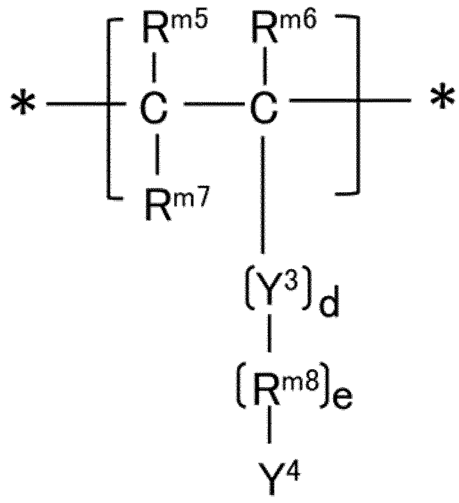

··· (3)

[In Formula (3), $R^{m5}$ to $R^{m7}$ are each independently a hydrogen atom or an alkyl group, $Y^3$ is an ether bond, an amide bond, or an ester bond; $R^{m8}$ is an alkylene group, a cycloalkylene group, an alkenylene group, an alkoxylene group, or an arylene group, and these groups may have a substituent; $Y^4$ is a hydrogen atom, an alkyl group, a hydroxy group, $Si(OH)_3$, $Si(R^{10})_3$, $NH_2$, $C(O)CH_3$, $C(O)NH_2$, or $N(COCH_3)$; d and e each independently represent an integer of 0 to 3; and * represents a single bond constituting the main chain of the polymeric compound].

Preferably, the polymer compound having a host group and the polymer compound having a guest group have the structural unit represented by the above formula (2) and, optionally, the structural unit represented by the above formula (3).

A terminal group ($R^E$) may be bonded to a terminal of the polymer compound having the structural unit represented by the above formula (2). The terminal group include an alkyl group having 1 to 10 carbon atoms or a group represented by $Si(R^{E1})_3$, where $R^{E1}$ is a hydrogen atom or an alkyl group having 1 to 10, 1 to 6, 1 to 3, or 1 to 2 carbon atoms, or an aryl group.

The polymer compound having the structural units represented by the above formula (2) and formula (3) may be a random copolymer, a block copolymer, or an alternating sequence copolymer, etc., and the order of the sequence of the constitutional units is not particularly limited.

The ratio of the structural unit in the polymer compound having the structural unit represented by the above formula (2) is not particularly limited. For example, with respect to the whole structural units (monomer units) constituting the polymer compound having the structural unit represented by the above formula (2), the ratio of the constitutional unit represented by the above formula (2) may be 0.01 mol% or more, 0.1 mol% or more, 1.0 mol% or more, 5 mol% or more, 10 mol% or more, or 15 mol% or more, and/or may be 90 mol% or less, 75 mol% or less, 50 mol% or less, 25 mol% or less, or 20 mol% or less.

Further, with respect to the whole constitutional units (monomer units) constituting the structural units represented by the above formula (2) and formula (3), the ratio of the structural unit represented by the above formula (2) may be 0.01 mol% or more, 0.1 mol% or more, 1.0 mol% or more, 5 mol% or more, 10 mol% or more or 15 mol% or more, and/or 30 mol% or less, 25 mol% or less or 20 mol% or less, and the content ratio of the constitutional unit represented by the above formula (3) is 70 mol% or more and 75 mol% or more or 80 mol% or more, and/or may be 99.99 mol% or less, 99.9 mol% or less, 99 mol% or less, 95 mol% or less, or 90 mol% or less.

In formula (2) above, each of $R^{m1}$ to $R^{m3}$ is independently a hydrogen atom or an alkyl group, preferably a hydrogen atom or an alkyl group having 1 to 12, 1 to 6 or 1 to 3 carbon atoms, and particularly preferably a hydrogen atom. $R^{m1}$ to $R^{m3}$ are preferably identical to each other.

In formula (2) above, $R^{m4}$ is preferably an alkylene group having 1 to 12, 1 to 6 or 1 to 3 carbon atoms, or an arylene group having 6 to 12 carbon atoms, and particularly preferably an alkylene group having 1 to 12, 1 to 6 or 1 to 3 carbon atoms.

In formula (2) above, $Y^2$ is preferably an ether bond, an amide bond, an ester bond, N(H), or N $(COCH_3)$, and particularly preferably an ether bond, an amide bond, or an ester bond.

In formula (2) above, "a", "b", and "c" are preferably each independently an integer of 0 to 2, and particularly

preferably 0 or 1.

In formula (3) above, each of $R^{m5}$ to $R^{m7}$ is independently a hydrogen atom or an alkyl group, preferably a hydrogen atom or an alkyl group having 1 to 12, 1 to 6 or 1 to 3 carbon atoms, and particularly preferably a hydrogen atom. $R^{m5}$ to $R^{m7}$ are preferably identical to each other.

In formula (3) above, $R^{m8}$ is preferably an alkylene group having 1 to 12, 1 to 6 or 1 to 3 carbon atoms, or an arylene group having 6 to 12 carbon atoms, and particularly preferably an alkylene group having 1 to 12, 1 to 6 or 1 to 3 carbon atoms.

In formula (3) above, $Y^4$ is preferably an alkyl group having 1 to 12, 1 to 6 or 1 to 3 carbon atoms, C (O) $CH_3$, C (O) $NH_2$, or N ($COCH_3$).

In formula (2) above, "d" and "e" are preferably each independently an integer of 0 to 2, and particularly preferably 0 or 1.

In one preferred embodiment, $R^{m1}$, $R^{m2}$, $R^{m3}$, $R^{m4}$, $Y^1$, "a", and "b" in Formula (2) above are the same as $R^{m5}$, $R^{m6}$, $R^{m7}$, $R^{m8}$, $Y^3$, "d", and "e" in (3) above, respectively.

In the above formula (1) and/or formula (2), the host group represented by $R^Y$ is preferably $\alpha$-cyclodextrin, $\beta$-cyclodextrin, or $\gamma$-cyclodextrin. The guest group represented by $R^Z$ is preferably an alkyl group which may have a substitute or an aryl group which may have a substituent.

$R^1$ to $R^5$ are each independently a hydrogen atom or an alkyl group, a cycloalkyl group, an alkenyl group, an alkoxy group, a cycloalkoxy group, a hydroxy group, a carboxyl group, an aldehyde group, an aryl group, or an aryloxy group, and are more preferably an alkyl group, a cycloalkyl group, an alkenyl group, an alkoxy group, a cycloalkoxy group, a hydroxy group, a carboxyl group, an aldehyde group, an aryl group, or an aryloxy group, and are particularly preferably an alkyl group, a cycloalkyl group, or an aryl group, and are most preferably an aklyl group. The alkyl group as $R^1$ to $R^5$ preferably has 1 to 10, more preferably 1 to 6, particularly preferably 1 to 3, and most preferably 1 to 2 carbon atoms.

In formula (1) and/or formula (2) described above, with regard to $R^Y$ and $R^Z$, reference may be made to the above description for the host group and guest group in the present disclosure.

In formula (1) and/or formula (2) above, $R^1$, $R^2$, $R^3$, $R^4$ and $R^5$ are preferably a methyl group or a phenyl group, and particularly preferably a methyl group. When $R^1$, $R^2$, $R^3$, $R^4$ and $R^5$ are a methyl group, solubility in a nonpolar solvent such as a hydrocarbon compound or silicone is further improved.

In formula (1) and/or formula (2) above, from the viewpoint of easier synthesis, $R^6$ is preferably a group represented by $-R^{R1}$-COOH or a group represented by $-R^{R2}$-COO-$R^{R3}$, and these groups may have a substituent, and $R^{R1}$, $R^{R2}$, and $R^{R3}$ are each an alkyl group or an alkylene group having 1 to 10 carbon atoms. Each of $R^{R1}$, $R^{R2}$, and $R^{R3}$ may be an alkyl group or an alkylene group having 1 to 8 carbon atoms, 1 to 6 carbon atoms, or 1 to 3 carbon atoms.

In particular, in formula (1) and/or formula (2) described above, $R^1$ to $R^5$ may be a methyl group, and/or $R^6$ may be a group represented by $-R^{R1}$-COOH or a group represented by - $R^{R2}$-COO-$R^{R3}$.

Cosmetic materials according to the present disclosure may bring about excellent physical properties such as improved durability, due to self-repairing property based on the host-guest interaction. Without wishing to be bound by theory, it is considered that, for example, when the cosmetic material according to the present disclosure is used as a cosmetic coating film, the polymer compounds reversibly re-bind via a host-guest interaction to close a scratch (including a minute scratch which cannot be visually observed) of the cosmetic coating film, and as a result, the durability of the cosmetic material is further improved.

In one preferred embodiment according to the present disclosure, in formula (1) and/or formula (2) above, k=0 and $R^1$ to $R^3$, $R^5$, and $R^6$ are an alkyl group. In this case, a cosmetic material having particularly excellent durability is obtained, which is preferable. Without wishing to be bound by theory, it is considered that when the Si atom constituting the main chain or a side chain of the polymer compound having a host group is modified with an alkyl group, the compatibility between the polymer compound having a host group and the polymer compound having a guest group is improved as compared with a case where the polymer compound is modified with a substituent having a relatively high polarity, leading to facilitation of the bonding (re-bonding) based on the host-guest interaction, so that the self-repairing property of the cosmetic material is further improved.

In particular, in formula (1) and/or formula (2) above:
k=0; $R^1$ to $R^3$ and $R^5$ are a methyl group; and $R^6$ is an alkyl group having two or more carbon atoms, more preferably an alkyl group having 4 or more carbon atoms, still more preferably is an alkyl group having 4 to 30 carbon atoms, 4 to 24 carbon atoms, 4 to 18 carbon atoms, 4 to 12 carbon atoms, or 4 to 8 carbon atoms, and especially a pentyl group. In this case, a cosmetic material having particularly excellent self-repairing property can be obtained.

In the above formula (1) and/or formula (2), from the viewpoint of easier synthesis, "p" and/or "q" are preferably 1 to 3, and more preferably 1 or 2, respectively.

Preferably, $X^1$ and $X^2$ are each independently Si $(OH)_2$, N (H), or N ($COCH_3$), an amide bond, or an ester bond, or a carbonyl group, an alkylene group, a cycloalkylene group, or an arylene group, and more preferably N (H) or N ($COCH_3$), an amide bond, or an ester bond, or an alkylene group.

$R^{10}$ is preferably a hydrogen atom or an alkyl group, cycloalkyl group, carboxyl group, aldehyde group, aryl group, more preferably a hydrogen atom or an alkyl group, a cycloalkyl group, an aryl group, and particularly preferably a hydrogen atom or an alkyl group. When $R^{10}$ is an alkyl group, it has preferably 1 to 10, more preferably 1 to 6, and particularly preferably 1 to 3 carbon atoms.
In formula (1) and/or formula (2) above, from the viewpoint of easier synthesis, $X^1$ is preferably an alkylene group having 1 to 10 carbon atoms, N $(COCH_3)$, an amide bond, or a carbonyl group. When $X^1$ is an alkylene group, it has preferably 1 to 6, more preferably 1 to 4, and particularly preferably 1 to 2 carbon atoms.
In formula (1) and/or formula (2) above, preferably, $(X^1)_p$ is $R^{P1}$-N$(COCH_3)$ or $(CH_2)_2$-N $(COCH_3)$. Further, in formula (1) and/or formula (2) above, particularly preferably, $-(X1)_p-R^Y$ is represented by $-R^{P1}$-N$(COCH_3)$-$R^Y$. $R^{P1}$ is an alkylene group which may have a substituent, and preferably has 1 to 10, more preferably 1 to 6, and particularly preferably 1 to 3 carbon atoms. $R^{P1}$ preferably does not have a substituent. Particularly preferably, in formula (1) and/or formula (2) above, $(X^1)_p$ is $(CH_2)_3$-N$(COCH_3)$.
From the viewpoint of easier synthesis, in formula (1) and/or formula (2) above, $X^2$ is preferably an alkylene group having 1 to 12 carbon atoms which may have a substituent, an amide bond, or a carbonyl group. When $X^2$ is an alkylene group, it has preferably 1 to 8, more preferably 1 to 6, and particularly preferably 1 to 3 carbon atoms.
In formula (1) and/or formula (2) above, $(X^2)_q$ is particularly preferably $-R^{11}$-CO-O- or - $R^{11}$-CO-N(H)-. Further, in formula (1) and/or formula (2) above, particularly preferably, $-(X^2)_q-R^Z$ is represented by-$R^{11}$ -CO-O-$R^Z$, or -CO-N (H)-$R^Z$. $R^{11}$ is an alkylene group having 1 to 12 carbon atoms which may have a substituent, and preferably has 1 to 10, more preferably 1 to 6, particularly preferably 2 to 4, and most preferably 3 carbon atoms. $R^{11}$ preferably does not have a substituent.
In formula (1) and/or formula (2) above, particularly preferably,

$(X^1)_p$ is $-(CH_2)_3$-N$(COCH_3)$-, and j is an integer of 1 or more,
and/or
$(X^2)_q$ is $-R^{11}$ -CO-O- or $-R^{11}$ -CO-N(H)-, wherein $R^{11}$ is an alkylene group having 1 to 12 carbon atoms which may have a substituent, and particularly an alkylene group having 3 to 6 carbon atoms, and k is an integer of 1 or more.

From the viewpoint of easier synthesis, in formula (1) and/or formula (2) above, when at least one of $X^1$ and $X^2$ is represented by $Si(R^{10})_2$, $R^{10}$ is preferably a hydrogen atom, a methyl group, or a phenyl group, particularly preferably a hydrogen atom or a methyl group, and most preferably a methyl group.
The value of "n" may be an integer of 5 or more, 10 or more, 20 or more, or 50 or more, and/or may be an integer of 1000 or less, 500 or less, 250 or less, 200 or less, 150 or less, or 100 or less.
With respect to formula (1) to (3) above, examples of the alkyl group include an alkyl group which may have a substituent, a linear, branched or cyclic alkyl group of C1 to C18. Specific examples thereof include an alkyl group such as methyl, ethyl, n-propyl, isopropyl, n-butyl, isobutyl, sec-butyl, tert-butyl, pentyl, cyclopentyl, hexyl, cyclohexyl, isohexyl, dodecyl, octadecyl, and adamantyl. Examples of the alkylene group include a linear, branched or cyclic alkylene group of C1 to C18, such as methylene, ethylene, n-propylene, isopropylene, n-butylene, and isobutylene.
With respect to formula (1) to (3) above, the aryl group includes an aryl group which may have a substituent, and specific examples thereof include an aryl group having a single ring or two or more rings, and specific examples thereof include phenyl, tolyl, xylyl, naphthyl, anthryl, and phenanthryl. Examples of the arylene group include an arylene group having a single ring or two rings, and a phenylene group.
With respect to formulas (1) to (3) above, the alkenyl group includes a linear or branched alkenyl group having 2 to 20 carbon atoms, such as vinyl, 1-propen-1-yl, 2-propen-1-yl, isopropenyl, 2-buten-1-yl, 4-penten-1-yl, and 5-hexen-1-yl.
With respect to formula (1) to (3) above, the alkoxy group includes an alkoxy group having 1 to 10 carbon atoms, for example, a methoxy group, an ethoxy group, a propoxy group, an isopropoxy group, a butoxy group, an isobutoxy group, a sec-butoxy group, a pentyloxy group, and a hexyloxy group.
With respect to formula (1) to (3) above, the substituent includes an alkyl group such as a methyl group and ethyl group, a halogen atom, a carboxyl group, an ester group, an amide group, and a hydroxy group.

(Ratio of each constituting unit which constitutes the polyorganosiloxane structure)

In formula (1) or formula (2) above, for each constitutional unit which is indicted by "h", "i", "j", and "k", respectively, the ratio of each constitutional unit $P_h$,$P_i$, $P_j$, and $P_k$ to the sum of all of the constitutional units constituting the structure represented by formula (1) or formula (2) described above can be defined as follows, respectively:

$$P_h=100*h/(h + i + j + k)$$

$$P_i=100^* i/(h+i+j+k)$$

$$P_j=100^* j/(h+i+j+k)$$

$$P_k=100^*k/(h+i+j+k).$$

$P_h$, $P_i$, $P_j$, and $P_k$ represent a ratio of the number of moles of each constitutional unit, which are each indicated by "h", "i", "j", or "k", to the total number of moles of all constitutional units constituting the structure represented by formula (1) or formula (2) described above.

The value of $P_h$ may be 70 mol% to 100 mol%, preferably 90 mol% to 100 mol%, more preferably 95 mol% to 98 mol%, and still more preferably 96 mol% to 97 mol%.

The value of $P_i$ may be 0 mol% to 30 mol%, preferably 0 mol% to 20 mol%, more preferably 0.5 mol% to 10 mol%, and still more preferably 1 mol% to 5 mol%.

The values of $P_j$ may be 0.01 mol% to 25 mol%, 0.01 mol% to 10 mol%, 0.1 mol% to 5 mol%, or 0.1 mol% to 2 mol%. Preferably, the value of $P_j$ is 0.1 mol% to 0.9 mol%, 0.2 mol% to 0.8 mol%, 0.3 mol% to 0.7 mol%, or 0.4 mol% to 0.6 mol%, in which case a membrane or a film-shaped cosmetic material may have particularly good elasticity.

The values of $P_k$ may be 0.01 mol% to 25 mol%, 0.01 mol% to 10 mol%, 0.1 mol% to 5 mol%, or 0.5 mol% to 3 mol%.

The values of $P_h$, $P_i$, $P_j$, and $P_k$ can be calculated from the amount of the charge of the raw materials used to produce each of the constituent units indicted by "h", "i", "j", and "k", respectively, and the $^1$H-NMR measurement data for the polymer compound.

(Positions of host group and guest group)

In one embodiment of the cosmetic material according to the present disclosure,

the polymer compound having a host group contained in the cosmetic material has the host group in a side chain of the polymer, and
the polymer compound having a guest group contained in the cosmetic material has the guest group in a side chain of the polymer.

In another embodiment of the cosmetic material according to the present disclosure,

the polymer compound having a host group contained in the cosmetic material has the host group in a side chain of the polymer, and
the polymer compound having a guest group contained in the cosmetic material has the guest group at a terminal of the polymer.

In still another embodiment of the cosmetic material according to the present disclosure,

the polymer compound having a host group contained in the cosmetic material has the host group at a terminal of the polymer and
the polymer compound having a guest group contained in the cosmetic material has the guest group in a side chain of the polymer.

(Polymer compound having no siloxane bond)

In one embodiment according to the present disclosure, the polymer compound having a host group or the polymer compound having a guest group contained in the cosmetic material does not include a siloxane bond. In this embodiment, a polymer compound containing no siloxane bond (hereinafter, also referred to as a "non-siloxane polymer compound") includes, for example, a polymer and a copolymer of at least one monomer selected from a vinyl compound, an acrylic compound, an olefin, a styrene, an acrylic ester, and a methacrylic ester; and a block copolymer containing these polymers and copolymers. Examples thereof include a vinyl resin, an acrylic resin, a urethane resin, an epoxy resin, a polyimide resin, a polyester resin, or a polycarbonate resin. These resins may have a substituent other than the host group and/or guest group. With regard to the polymer compound having a guest group and/or host group and

containing no siloxane bond, reference can be made to the description of Patent Document 2.

(Combination of polymer compound contained in cosmetic material)

For example, the cosmetic material according to the present disclosure may have polymer compounds of at least one of the following (i) to (iv):

(i) A non-siloxane polymer compound having a host group, and a siloxane polymer compound having a guest group;
(ii) A siloxane polymer compound having a host group, and a non-siloxane polymer compound having a guest group;
(iii) A siloxane polymer compound having a host group, and a siloxane polymer compound having a guest group;
(iv) A siloxane polymer compound having a host group and a guest group.

(Ratio of host and guest)

When the cosmetic material contains a polymer compound having a host group and a polymer compound having a guest group, the respective content ratios in the cosmetic material are not particularly limited. For example, the content of the polymer compound having a host group may be 10 to 90 % by mass, and the content of the polymer compound having a guest group may be 90% by mass to 10% by mass, based on the total of the polymer compound having a host group and the polymer compound having a guest group.

The ratio of the amount of the host group and the amount of the guest group contained in the cosmetic material is not particularly limited. The molar ratio of the host group and the guest group contained in the cosmetic material may be from 0.1:1 to 1:0.1. Alternatively, in the cosmetic material, the polymer compound having a guest group may be contained in an amount of 1 parts by mass or more, 10 parts by mass or more, or 20 parts by mass or more, and/or may be contained in an amount of 1000 parts by mass or less, 250 parts by mass or less, or 100 parts by mass or less, based on 100 parts by mass of the polymer compound having a host group.

<<Preparation of polymer compound>>

(Preparation of polymer compound: non-siloxane polymer compound having host group)

The polymer compound having a host group and containing no siloxane bond (i.e., the non-siloxane polymer compound having a host group) can be prepared by a known method, and can be prepared, for example, by a method described in Patent Document 2.

(Preparation of polymer compound: non-siloxane polymer compound having guest group)

The polymer compound having a guest group and containing no siloxane bond (i.e., the non-siloxane polymer compound having a guest group) can be prepared by a known method, and can be prepared, for example, by a method described in Patent Document 2.

(Preparation of polymer compound: polymer compound having host group and containing siloxane bond)

The siloxane polymer compound having a host group can be prepared, for example, by a method comprising the following steps:

(i) a step of providing a polymer, in which a polymer compound having a siloxane bond (a siloxane polymer compound) is provided,
(ii) a step of providing a host group precursor, in which a host group precursor compound is provided,
(iii) a step of host group addition reaction, in which the siloxane polymer compound and the host group precursor compound are reacted in the presence of a metal catalyst to obtain a siloxane polymer compound having a host group.

(Step of providing a polymer)

In the step of providing a polymer, a polymer compound having a siloxane bond (siloxane polymer compound) is provided. The polymer compound having a siloxane bond is preferably a polyorganosiloxane. The polymer compound having a siloxane bond preferably has a structure suitable for reacting with the host group precursor compound, and has, for example, a hydrogen atom directly bonded to Si.

The siloxane polymer compound provided in the step of providing a polymer may be for example an SiH-containing silicone, and specifically, for example, a polymer having a structure represented by the following formula (4):

[Chem 7]

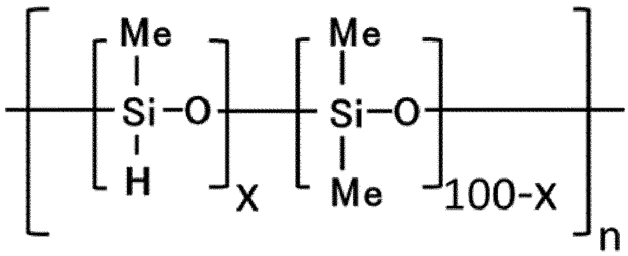 ···(4)

[In formula (4), x represents an integer of 1 or more; n represents an integer of 1 or more].

In formula (4) above, "x" may be 2 or more, 5 or more, 10 or more, or 20 or more, and/or may be 95 or less, 90 or less, 75 or less, or 50 or less. In formula (4) above, "n" may be 5 or more, 10 or more, or 50 or more, and/or may be 1000 or less, 500 or less, 250 or less, 100 or less, or 75 or less.

(Step of providing a host group precursor)

In the step of providing a host group precursor, a host group precursor compound is provided. The host group precursor compound preferably has a linker structure for binding to a polymer compound having a siloxane bond. Examples of the host group precursor compound include a cyclodextrin having a linker structure. Examples of the linker structure include a vinyl group, and a particular example thereof include a group represented by the following formula ($X_L$):

$$C(H)_2=C(H)-C(H)_2-N(COCH_3)-* \cdots(X_L)$$

[In formula ($X_L$), * represents a single bond to a cyclodextrin.]

(Step of host group addition reaction)

In the step of host group addition reaction, the siloxane polymer compound and the host group precursor compound are reacted in the presence of a metal catalyst to obtain a siloxane polymer compound having a host group. For example, a siloxane polymer compound having a hydrogen atom directly bonded to Si of the main chain of the polymer may be reacted with a cyclodextrin having a vinyl group as a linker structure in the presence of a metal catalyst, in order to obtain a siloxane polymer compound in which the cyclodextrin is bonded to the Si atom of the main chain of the polymer via the linker structure.

Preferably, after the reaction for adding the host group, the hydrogen atoms directly bonded to the Si atoms in the polymer compound are substituted. This further improves the chemical stability of the resulting cosmetic material. Examples of the compound usable for this substitution include an acrylic ester or an alkene having two or more carbon atoms. The alkene having two or more carbon atoms is preferable because the resulting cosmetic material has particularly excellent self-repairing property. Among the alkenes having two or more carbon atoms, an alkene having 5 or more carbon atoms is more preferable, and an alkene having 5 to 30 carbon atoms, 5 to 24 carbon atoms, 5 to 18 carbon atoms, 5 to 12 carbon atoms, or 5 to 8 carbon atoms is further preferable, and 1-pentene is particularly preferable.

(Metal catalyst)

Examples of the metal catalyst used in the step of host group addition reaction described above include platinum (Pt).

(Preparation of a polymer: Preparation of polymer compound having guest group and containing siloxane bond)

The siloxane polymer compound having a guest group can be produced, for example, by a method comprising the following steps:

(i) a step of providing a polymer, in which a polymer compound having a siloxane bond (siloxane polymer compound) is provided,

(ii) a step of providing a guest group precursor, in which a guest group precursor compound is provided,
(iii) a reaction step, in which the siloxane polymer compound and the guest group precursor compound are reacted in a solvent to obtain a siloxane polymer compound having a guest group.

(Step of providing a polymer)

In the step of providing a polymer, a polymer compound having a siloxane bond having a carboxyl group is provided. The polymer compound having a siloxane bond is preferably a polyorganosiloxane. The polymer compound having a siloxane bond, which is provided in the step of providing a polymer, preferably has a structure suitable for reacting with the guest group precursor compound so as to form a siloxane polymer compound having a guest group, and has, for example, a carboxyl group which is bonded to Si of the main chain of the polymer via an alkylene group having 1 to 12 carbon atoms.

(Step of providing a guest group precursor)

In the step of providing a guest group precursor, a guest group precursor compound is provided. The guest group precursor compound preferably has a linker structure for binding to the polymer compound having a siloxane bond. Specific examples of the guest group precursor compound include an adamantyl having a linker structure. Examples of the linker structure include an amino group.

(Reaction step)

In the reaction step, the siloxane polymer compound and the guest group precursor compound are reacted in a solvent to obtain a siloxane polymer compound having a guest group. For example, a siloxane polymer compound having a carboxyl group bonded to Si of the main chain of the polymer via an alkyl group may be reacted with an adamantyl having an amino group as a linker structure, in a solvent in the presence of a condensing agent, in order to obtain a siloxane polymer compound having a guest group. In this case, the siloxane polymer compound having a guest group has a structure in which the adamantyl is bonded via a linker structure to Si atom constituting the main chain of the siloxane polymer compound. Examples of the condensing agent include 1-hydroxybenzotriazole (HOBt; Tokyo Chemical Industry Co., Ltd., H0468, Cas:80029-43-2) and N,N'-dicyclohexylcarbodiimide (DCC; Nacalai Tesque, INC., 11913-52, Cas:538-75-0).

(Preparation of polymer compound: siloxane polymer compound having host group and guest group)

The siloxane polymer compound having a host group and a guest group can be obtained, for example, by polymerizing a siloxane polymer compound having a host group, which is prepared by the above method, and a siloxane polymer compound having a guest group, which is produced by the above method, in order to form a block copolymer.

(Preparation of polymer compound: Preparation of polymer compound having host group and/or guest group and siloxane bond)

The polymer compound having a host group and/or guest group and a siloxane bond can also be prepared, for example, by subjecting a monomer represented by the following formula (5) and a monomer represented by the formula (6) to a polymerization reaction. The specific embodiment of the polymerization reaction is not particularly limited, and a known method can be used.

[Chem 8]

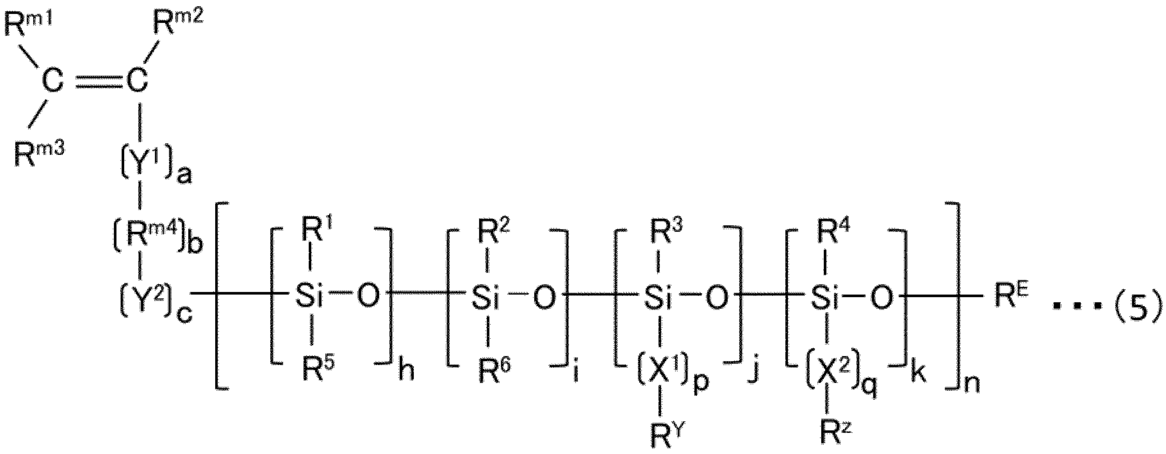

[Chem 9]

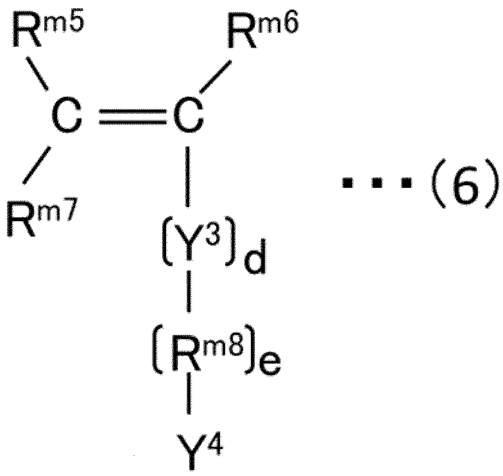

For each symbol in formula (5) and formula (6) described above, reference may be made to the description of each symbol in formula (2) and formula (3) described above. Note that $R^E$ represents a terminal group. With regard to $R^E$, it is also possible to refer to the above-mentioned descriptions.

(Cross-linking)

In one embodiment of the cosmetic material according to the present disclosure, at least one of the polymer compound having a host group and the polymer compound having a guest group, or the polymer compound having a host group and a guest group, is crosslinked. The cross-link may be, for example, a cross-link through a bond other than the host-guest interaction, such as a cross-link through a covalent bond (e.g., siloxane cross-linking).

For example, the polymer compound having a host group may form a host body by being cross-linked, and/or the polymer compound having a guest group may form a guest body by being cross-linked.

As for a method for cross-linking a polymer compound and as for a cross-linking agent, a known method and a known agent may be used, depending on the type of the polymer compound. The cross-link may be formed, for example, by light irradiation. Examples of the cross-linking agent include N,N'-methylenebisacrylamide (MBAAm), and ethylene glycol dimethacrylate (EDMA).

<<Manufacture of cosmetic material>>

The cosmetic material according to the present disclosure can be produced, for example, by individually preparing a polymer compound having a host group and a polymer compound having a guest group, respectively, and

mixing the obtained polymer compound having a host group and polymer compound having a guest group. When mixing the polymer compound having a host group and the polymer compound having a guest group, for example, they may be mixed in a state in which both of them are solid; they may be mixed in a state in which one or both of them is liquid; or they may be mixed in a state in which one or both of them is in a solution. It is also possible that a polymer compound of a solid state is added to a solution of the other polymer compound.

The condition for mixing the polymer compound having a host group and the polymer compound having a guest group is not particularly limited. For example, suitable temperature at the time of mixing, mixing time, and a means for mixing, etc., are selected accordingly.

In one embodiment of the cosmetic material according to the present disclosure, the cosmetic material is in the form of a paste at room temperature. By selecting a polymer compound which is in the form of a paste at room temperature as the polymer compound contained in a cosmetic material, a cosmetic material in the form of a liquid can be obtained. Alternatively, a liquid cosmetic material may be obtained by dissolving a polymer compound in a solvent. A cosmetic material which is liquid at room temperature is particularly preferred because it can be relatively easily formed in to a film.

Examples of the polymer compound which is liquid at room temperature include a polymer compound having a polyorganosiloxane in the main chain thereof, in particular a dimethylpolysiloxane.

The solvent for dissolving the polymer compound is not particularly limited, and examples thereof include silicone oil and hydrocarbon oil. Examples of the silicone oil include one or two or more selected from octamethylcyclotetrasiloxane, decamethylcyclopentasiloxane, dodecamethylcyclohexasiloxane, dimethylpolysiloxane, and caprylylmethicone, and more preferably one or two or more selected from decamethylcyclopentasiloxane and dimethylpolysiloxane, and further preferably dimethylpolysiloxane having a degree of polymerization of 10 or less. Examples of the hydrocarbon oil include toluene and isododecane, and isododecane is preferred.

The polymer compounds contained in the cosmetic material according to the present disclosure may be bonded to each other by the host-guest interaction, or may not be bonded to each other by the host-guest interaction.

The cosmetic material can be formed into a film. If the cosmetic material is in the form of a film, it can be used as a cosmetic coating film applied on the skin, etc. When the cosmetic material is in the form of a film, there is no particular limitation on the thickness thereof, and an appropriate thickness can be selected depending on the application. For example, the cosmetic material may be adjusted to 1 nm to 1 cm, and, from the viewpoint of better film formability, may be adjusted to 1 $\mu$m to 100 $\mu$m.

(Shaping)

A method for shaping the cosmetic material as a cosmetic coating film is not particularly limited. For example, the cosmetic material can be shaped into a cosmetic coating film by preparing a solution or dispersion of a polymer compound and by using this solution or dispersion for coating.

The method for producing a cosmetic material may comprise, for example, the following steps:

preparing a host solution by dissolving a polymer compound having a host group in a solvent and
producing a cosmetic material A by adding a polymer compound having a guest group to this host solution.

In the above method, alternatively, a cosmetic material A may be prepared by:

dissolving a polymer compound having a guest group in a solvent to prepare a guest solution, and
adding a polymer compound having a host group to the guest solution.

The step of shaping the cosmetic material A prepared as described above into a cosmetic coating film may comprise, for example, the following step:
applying the cosmetic material A onto a target object with a finger and drying it, in order to form a cosmetic coating film.

In another embodiment of the present disclosure, a method of manufacturing a cosmetic material may comprise, for example, the following steps:
preparing a cosmetic material B by mixing a polymer compound having a host group, which is in the form of a paste or liquid, and a polymer compound having a guest group, which is in the form of a paste or liquid.

The step of shaping the cosmetic material B prepared as described above into a cosmetic coating film may comprise, for example, the following step:
applying the cosmetic material B to a target object to form a cosmetic coating film.

Incidentally, in the method for manufacturing the cosmetic material B described above, it is also possible that only one of the polymer compound having a host group and the polymer compound having a guest group is in a paste state or a liquid state.

<<Solvent>>

In addition to the components described above, the cosmetic material according to the present disclosure may further contain a solvent. By further containing a solvent, it is possible to provide a cosmetic material which is particularly excellent in coatability onto skin, etc.

The solvent which can be contained in the cosmetic material according to the present disclosure is not particularly limited, and examples thereof include water, alcohols, silicones, a hydrocarbon compound, an ester compound, ethers, and wax.

The alcohols include glycerin, butylene glycol (BG), propylene glycol (PG), dipropylene glycol (DPG), polyethylene glycol (PEG), ethanol, and oleyl alcohol.

Examples of the silicones include dodecamethylcyclohexasiloxane, octamethylcyclotetrasiloxane, decamethylcyclopentasiloxane, dimethylpolysiloxane, caprylylmethicone, methylpolysiloxane, methylphenylpolysiloxane, cyclopentasiloxane, copolymer of aminoethylaminopropylmethylsiloxane and dimethylsiloxane, aminopropyldimethicone, methyltrimethicone, tris(trimethylsilyl) methylsilane, tetrakis (trimethylsilyl) silane, amodimethicone, cyclomethicone, and phenyltrimethicone. Among these, 1 or 2 or more selected from decamethylcyclopentasiloxane and dimethylpolysiloxane are more preferred, and dimethylpolysiloxane having a degree of polymerization of 10 or less is further preferred.

Examples of the hydrocarbon compound include hydrogenated polyisobutene, petrolatum, mineral oil, squalane, paraffin, isoparaffin, alkyl benzoate, polyisobutene, isododecane, isotridecane, and isohexadecane light isoparaffin, and isododecane is preferable.

Examples of the ester compound include isopropyl palmitate, ethylhexyl palmitate, isopropyl myristate, and octyldodecyl myristate.

The ethers include ethyl perfluoro-butylether.

When the cosmetic material according to the present disclosure further has a solvent, the solvent is particularly preferably volatile. When the cosmetic material according to the present disclosure contains a volatile solvent, it is possible to easily form a cosmetic coating film, since the cosmetic material becomes a film through volatilization of the solvent when the cosmetic material is applied to the skin, etc.

The volatile solvent includes the alcohols described above; silicones such as dimethylpolysiloxane, methyltrimethicone, octamethylcyclotetrasiloxane, and decamethylcyclopentasiloxane, dodecamethylcyclohexasiloxane, tris (trimethylsilyl) methylsilane, and tetrakis (trimethylsilyl) silane; a hydrocarbon compound such as isododecane, isotridecane, isohexadecane, light isoparaffin, and hydrogenated polyisobutene; and ethers such as ethyl perfluorobutyl ether.

<<Other additives>>

The cosmetic material according to the present disclosure can contain each main component other than the above-mentioned components to the extent that the effect of the present invention is not impaired; examples thereof include a lower alcohol, a higher alcohol, a spherical powder, a film-forming agent, a refreshing agent, an oily component, a detergent, an aqueous component, a water-soluble polymer, an ultraviolet absorber, a humectant, anti-browning agent, an antioxidant, an antifoaming agent, a cosmetic component, a preservative, a metal ion sealing agent, a blood-circulation accelerant, various extraction liquids, an inorganic pigment, an organic pigment, a mineral, a dye such as an organic dye, a coloring agent, a thickener, a pH adjusting agent, a cold feeling agent, an antiperspirant, a disinfectant, a skin activator, and a perfume, which are able to be appropriately contained in the cosmetic material in order to impart various effects.

The lower alcohol is commonly used in cosmetic material as a usability modifier and a preservative aid. Examples of the lower alcohol include ethanol and isopropyl alcohol.

Examples of the spherical powder include an inorganic powder such as spherical silicic anhydride, spherical polymethyl methacrylate, spherical cellulose, spherical nylon, spherical polyethylene, and spherical silicone powder.

When a film-forming agent is additionally included, examples of the film-forming agent include polyvinyl alcohol, polyvinylpyrrolidone, polyvinyl acetate, and a latex such as alkyl polyacrylate, dextrin, and a cellulose derivative such as alkyl cellulose and nitrocellulose.

Examples of the refreshing agent include menthol derivatives, camphor derivatives, and essential oil.

EXAMPLES

Hereinafter, the invention according to the present disclosure will be described more specifically by way of Examples.

<<Measuring method>>

The measurements were performed by the following methods.

<$^1$H-NMR >

Nuclear magnetic resonance (NMR) spectra were recorded using ECA500 (JEOL, Ltd.) as an instrument. Measurements were performed at 25°C. Deuterated chloroform was used as the solvent. NMR chemical shifts were recorded in ppm (parts per million) relative to the residual solvent peak at 7.26 ppm (chloroform).

(IR)

Fourier-transform infrared spectra were measured by a FT/IR 6100 spectrometer (JASCO Corporation) in the range of 500 $cm^{-1}$ to 4000 $cm^{-1}$.

(MALDI-TOF MAS)

MALDI-TOF MS spectra were measured using autoflex maX LRF (Bruker) as an instrument.

<Elemental analysis>

Elemental analysis was performed using an elemental analyzer (manufactured by Yanako Analytic Systems Inc., CHN coder) based on the differential thermal conductivity method.

<<Preparation of compound>>

[Chem 10]

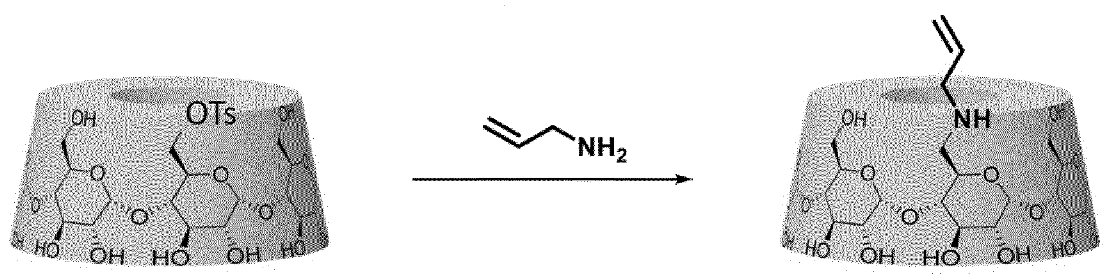

Compound 0 Compound 1

<Production example 1: Preparation of compound 1>

Compound 1 was prepared according to the following method:
Compound 0 (β-cyclodextrin-OTs; C-6 monotosylate d-β-CD) was prepared according to the process described in Tetrahedron Letters, Vol. 25, No. 31, 3331-3334, 1984. A 30.0 g (23.28 mmol) of Compound 0 was dissolved in 300 g (5.25 mol) of allylamine, and then refluxed overnight. Then, the reaction solution was dried under reduced pressure on an evaporator to obtain a dry matter. The resulting dry matter was dissolved by adding it to 600 mL of acetonitrile. Then, the precipitate in the solution was collected by suction filtration, and dissolved by adding it to 600 mL of acetonitrile. Then, the precipitate in the solution was collected by suction filtration and dried under reduced pressure in a vacuum oven at 100 °C overnight to obtain Compound 1.

The production of obtained compound 1 was confirmed by MALDI-TOFMS, $^1$H-NMR, and elemental analyses.

[Chem 11]

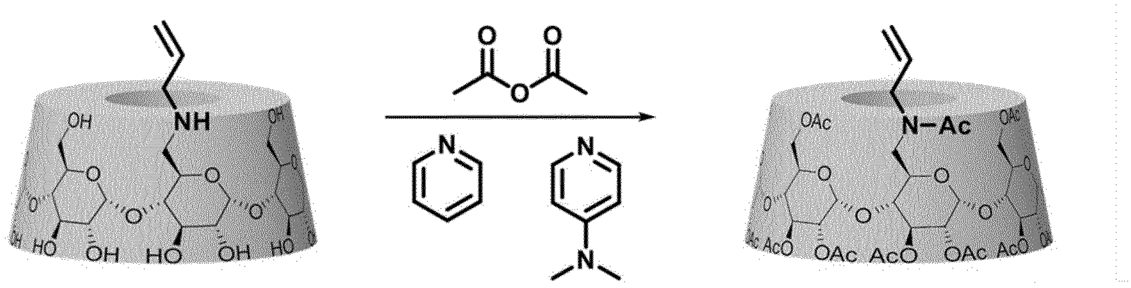

Compound 1 Host precursor compound 2

<Production example 2: Preparation of host precursor compound 2>

Host precursor compound 2 was prepared according to the following method:
To 20 g (17.0 mmol) of compound 1 obtained by the above method, 300 mL (300g, 3.8 mol) of dry pyridine and 170 mL (184 g, 1.8 mol) of acetic anhydride were added, and the mixture was stirred at 70°C overnight. Then, 60 mL of methanol was added dropwise while cooling the reaction solution with ice. Then, the reaction solution was dried under reduced pressure on an evaporator to obtain a dry matter. The resulting dry matter was dissolved in 100 mL of acetone and added dropwise to 2 L of water. Then, the precipitate in the solution was collected by suction filtration, and a solution obtained by dissolving the precipitate in 100 mL of acetone was added dropwise to 1.5 L of water. Then, the precipitate in the solution was collected by suction filtration and dried in a vacuum oven at 70 °C for one day under reduced pressure to obtain host precursor compound 2.

The production of host precursor compound 2 was confirmed by MALDI-TOFMS, $^1$H-NMR, and elemental analyses.

[Chem 12]

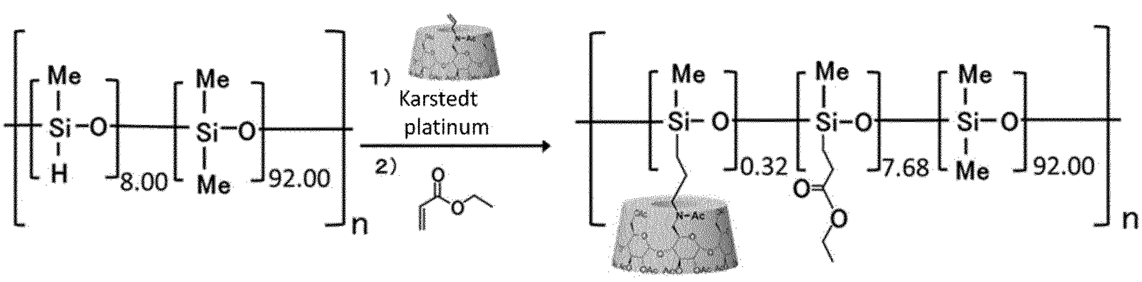


Polymer compound H1

<Production example 3: Preparation of Polymer compound HI having host group>

Polymer compound HI having a host group was prepared according to the following method:
A 1.8 g (0.9 mmol) of host precursor compound 2 was dissolved in 400 ml of toluene, and after nitrogen substitution, it was stirred at 105 °C. To this solution, a solution prepared by adding 12.0 g (14.4 mmol) of methyl hydrogen polysiloxane (kinematic viscosity 20 mm$^2$/s, effective hydrogen content 7.5 mol%) and 165 $\mu$L of a karstedt platinum solution in 400 ml of toluene was added dropwise over a period of 1 hour. Incidentally, the karstedt platinum solution was prepared using a platinum(0)-1,3-divinyltetramethyldisiloxane complex (19.0 % to 21.5 % as Pt, manufactured by TCI, P2075, Cas:68478-92-2, Product Code :P2075). The weight of Pt used was 28.5 mg. Four hours after completion of the dropwise addition, 4.8 g (48 mmol) of ethyl acrylate (Cas: 140-88-5, manufactured by Tokyo Chemical Industry Co., Ltd.) was added by a syringe. Of the obtained reaction solution, 10 g thereof was dissolved in 114 ml of isododecane (Marukasol R, Maruzen Petrochemical Co., Ltd.). The remaining unreacted CD (0.499 g) was removed by extraction into ethyl acetate, in order to obtain polymer compound HI having a host group, which was dissolved in isododecane.

The production of obtained polymer compound HI was confirmed by analysis of $^1$H-NMR and IR spectra.

For obtained polymer compound HI, the host group introduction rate measured by $^1$H-NMR was 0.32 mol%.

<Production example 4: Preparation of polymer compound H2 having host group>

Polymer compound H2 having a host group was prepared in the same manner as in the preparation of polymer compound HI having a host group described above, except that 0.6 g (0.3 mmol) of host precursor compound 2, 4.0 g (4.8 mmol) of methyl hydrogen polysiloxane, and 55 μL of karstedt platinum solution were used.

For obtained polymer compound H2, the host group introduction rate measured by $^1$H-NMR was 0.31 mol%.

<Production example 5: Preparation of polymer compound H3 having host group>

Polymer compound H3 having a host group was prepared in the same manner as in the preparation of polymer compound HI having a host group described above, except that 4.0 g (4.8 mmol) of host precursor compound 2, 4.0 g (4.8 mmol) of methyl hydrogen polysiloxane, and 55 μL of a karstedt platinum solution were used, and that the solvent amount was adjusted so that the concentration of the main chain silicone was 34.3 mmol.

For obtained polymer compound H3, the host group introduction rate measured by $^1$H-NMR was 0.20 mol%.

<Production example 6: Preparation of polymer compound H4 having host group>

Polymer compound H4 having a host group was prepared in the same manner as in the preparation of polymer compound HI having a host group described above, except that 2.4 g (1.2 mmol) of host precursor compound 2, 16.0 g (19.2 mmol) of methyl hydrogen polysiloxane, and 220 μL of karstedt platinum solution were used.

For obtained polymer compound H4, the host group introduction rate measured by $^1$H-NMR was 0.37 mol%.

<Production example 7: Preparation of polymer compound H5 having host group>

Polymer compound H5 having a host group was prepared in the same manner as in the preparation of polymer compound HI having a host group described above, except that 0.6 g (0.3 mmol) of host precursor compound 2, 2.0 g (2.4 mmol) of methyl hydrogen polysiloxane, and 55 μL of karstedt platinum solution were used.

For obtained polymer compound H5, the host group introduction rate measured by $^1$H-NMR was 1.0 mol%.

<Production example 8: Preparation of polymer compound H6 having host group>

Polymer compound H6 having a host group was prepared in the same manner as in the preparation of polymer compound HI having a host group described above, except that 0.6 g (0.3 mmol) of host precursor compound 2, 4.0 g (4.8 mmol) of methyl hydrogen polysiloxane, and 55 μL of karstedt platinum solution were used.

For obtained polymer compound H6, the host group introduction rate measured by $^1$H-NMR was 0.5 mol%.

[Chem 13]

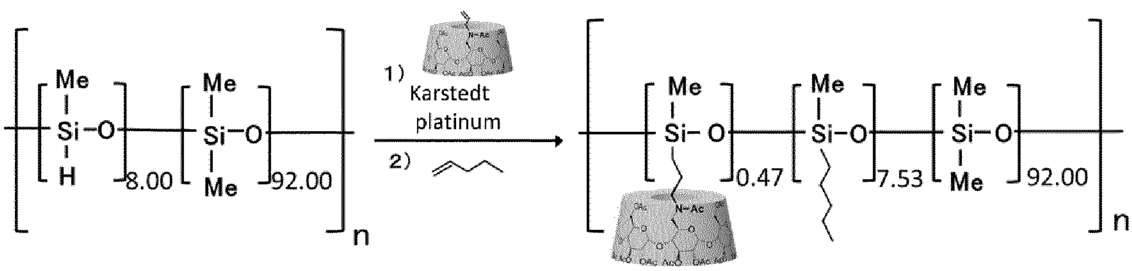


Polymer compound H7

<Production example 9: Preparation of polymer compound H7 having host group>

Polymer compound H7 having host group was prepared according to the following method:
A 1.8 g (0.9 mmol) of host precursor compound 2 was dissolved in 900 ml of toluene, and after nitrogen substitution, it was stirred at 105 °C. To this solution, a solution prepared by adding 12.0 g (11.48 mmol) of methyl hydrogen polysiloxane

and 165.0 μL of Karstedt platinum solution in 250 mL of toluene was added dropwise over a period of 30 minutes. Incidentally, the Karstedt platinum solution was prepared using a platinum(0)-1,3-divinyltetramethyldisiloxane complex (19.0 % to 21.5 % as Pt, manufactured by TCI, P2075, Cas:68478-92-2 Product Code :P2075). The weight of Pt used was 28.2 mg and the reagent concentration in the reaction solution was [Pt] = 0.09 mM. After 4 hours from the completion of the dropwise addition, 2.0 g (29.0 mmol) of 1-Pentene (manufactured by Tokyo Chemical Industry Co., Ltd.) was added. Reflux was carried out overnight, and toluene was distilled off by an evaporator, and then dissolved in hexane, and the precipitate was removed by a centrifuge, in order to obtain polymer compound H7 having a host group, which was dissolved in hexane.

The preparation of obtained polymer compound H7 was confirmed by the analysis of $^1$H-NMR and IR spectra.

For obtained polymer compound H7, the host group introduction rate measured by $^1$H-NMR was 0.47 mol%.

<Production example 10: Preparation of polymer compound H8 having host group>

Polymer compound H8 having a host group was prepared in the same manner as in the preparation of polymer compound H7 having a host group described above, except that 3.6 g (1.8 mmol) of host precursor compound 2 was used. The host group introduction rate of the polymer compound H8 was 0.63 mol%.

<Production example 11: Preparation of polymer compound G1 having guest group>

[Chem 14]

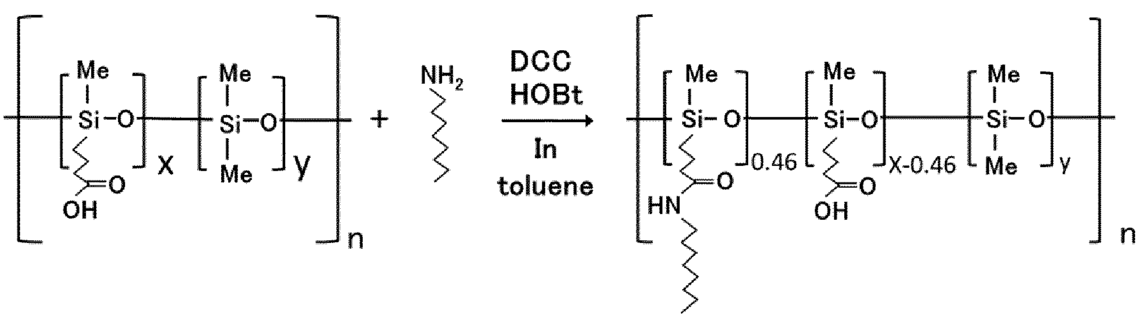


Polymer compound G1

[wherein, x and y are an integer of 0 or more, and x + y = 100.]

Polymer compound G1 was prepared according to the following method:
A 10.0g (1.3 mol) of side-chain type carboxyl-modified silicone oil (manufactured by Shin-Etsu Silicone Co., Ltd., X-22-3701E, carboxyl group equivalent = 4000 g/mol) was dissolved in 100 mL of toluene to obtain solution 1. Then, to this solution 1, 68.24 mg (0.5 mol) of HOBt was added to obtain solution 2. Then, 104.19 mg (0.5 mol) of DCC dissolved in 50 mL of toluene was added to this solution 2 to obtain solution 3. Then, 65.26 mg (0.5 mmol) of N-octylamine (Nacalai Tesque, Inc., 25512-72, Cas:111-86-4) was dispersed in 50 mL of toluene and added to solution 3 to obtain solution 4. This solution 4 was stirred overnight to obtain a clear supernatant and a white precipitate. The white precipitate was removed by filtration to give a clear filtrate. The clear filtrate was washed with saturated aqueous sodium bicarbonate and dehydrated with anhydrous sodium sulfate. Toluene was removed in an evaporator to obtain a colorless transparent oil. This colorless transparent oil was dried in vacuum at 85 °C overnight, in order to obtain polymer compound G1 having a guest group.

The production of obtained polymer compound G1 was confirmed by $^1$H-NMR (500 MHz) measurement.

For obtained polymer compound G1, the guest group introduction rate measured by $^1$H-NMR was 0.46 mol%.

<Production example 12: Preparation of Polymer compound G1' having guest group>

Polymer compound G1' was obtained in a similar way as Production example 11. The guest group introduction rate in obtained polymer compound G1' was 0.51 mol%.

[Chem 15]

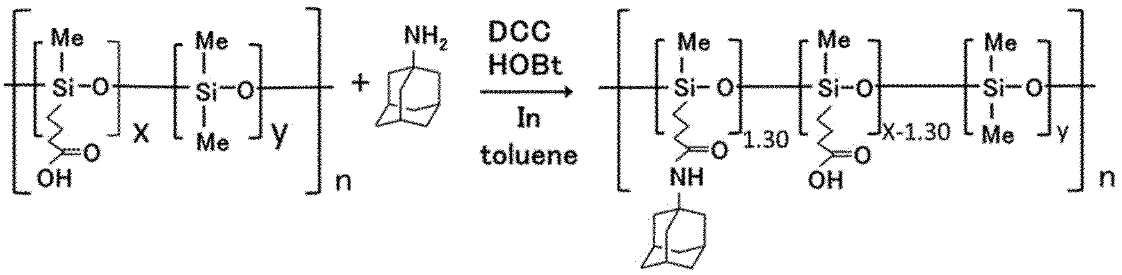


Polymer compound G2

[wherein, x and y are an integer of 0 or more, and x + y = 100.]

<Production example 13: Preparation of Polymer compound G2 having guest group>

Polymer compound G2 having a guest group was prepared in the same manner as in the preparation of polymer compound G1 having a guest group described above, except that 10000 mg (1.3mol) of side-chain type carboxyl-modified silicone oil and 95.7 mg (0.63 mmol) of 1-adamantylamine (manufactured by Fujifilm Waco Pure Chemical Corporation) were used instead of N-octylamine.

For obtained polymer compound G2 having a guest group, the guest group introduction rate measured by $^1$H-NMR was 1.3 mol%.

<Production example 14: Preparation of polymer compound A2>

[Chem 16]

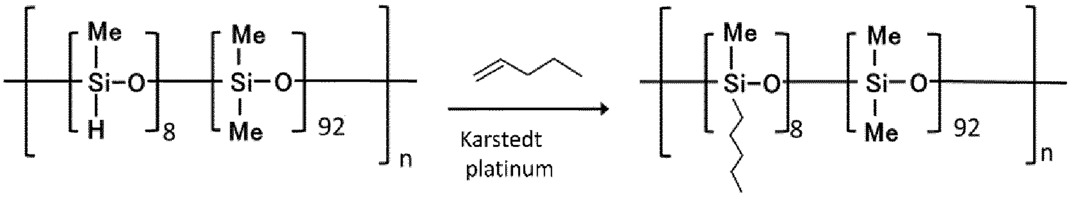


Polymer compound A2

Methyl hydrogen polysiloxane and karstedt platinum solutions, and 1-Pentene (manufactured by Tokyo Chemical Industry Co., Ltd.) were used to produce polymer compound A2.

<Production Example 15: Preparation of polymer compound CH-4 having host group>

Polymer compound CH-4 having a polyethyl acrylate main chain and $\gamma$-cyclodextrin as a host group was prepared, according to the method described in Macromolecules 2019, 52(7), 2659-2668. Incidentally, the preparation was carried out so that the unit having a host group in the obtained polymer compound was 1.0 mol%.

<<Example 1 (Ex.1)>>

The cosmetic material according to Example 1 was produced by dissolving polymer compound H5 having a host group prepared as in Production Example 7 described above and polymer compound G1 having a guest group prepared as in Production Example 11 described above in isododecane, and by adding additional compounds shown in Table 1 below in the amount shown in Table 1.

Polymer compound H5 and polymer compound G1, which have the polymethylsiloxane main chain, exhibited good solubility in isododecane which was used as a solvent.

The cosmetic material according to Example 1 was applied to the cheek with a finger to form a cosmetic coating film. Evaluations were performed for the following items:

(a) Tightness
(b) Close adhesiveness
(c) Resilience
(d) Durability.

<Tightness>

Tightness was evaluated according to the following three criteria:

| (Evaluation): | (Criteria) |
|---|---|
| Good: | There was no feeling of tightness. |
| Moderate : | There was a little feeling of tightness. |
| Poor : | There was feeling of tightness. |

<Close adhesiveness>

Close adhesiveness was evaluated according to the following three criteria:

| (Evaluation): | (Criteria) |
|---|---|
| Very good: | There was particularly good feeling of close adhesiveness. |
| Good : | There was feeling of close adhesiveness. |
| Poor : | There was no feeling of close adhesiveness. |

<Resilience>

The feeling of resilience was evaluated according to the following three criteria:

| (Evaluation): | (Criteria) |
|---|---|
| Good: | There was feeling of resilience. |
| Moderate: | There was a little feeling of resilience. |
| Poor: | There was no feeling of resilience. |

<Durability>

The evaluation of durability was conducted according to the following three criteria:

| (Evaluation): | (Criteria) |
|---|---|
| Good: | Durability was good. |
| Moderate: | Durability was moderate. |
| Poor: | Durability was poor. |

The evaluation results of Example 1 are shown in Table 2.

<<Example 2 (Ex.2)>>

The cosmetic material according to Example 2 was produced by dissolving polymer compound H5 having a host group prepared as in Production Example 7 described above and polymer compound G2 having a guest group prepared as in Production Example 13 described above in isododecane, and by adding additional compounds shown in Table 1 below in the amount shown in Table 1.

Polymer compound H5 and polymer compound G2, which have the polymethylsiloxane main chain, exhibited good solubility in isododecane which was used as a solvent.

The cosmetic material according to Example 2 was evaluated for the feeling of use in the same manner as in Example 1. Table 2 shows the results.

<<Example 3 (Ex.3)>>

The cosmetic material according to Example 3 was produced by dissolving polymer compound H7 having a host group prepared as in Production Example 9 described above and polymer compound G1 having a guest group prepared as in Production Example 11 described above in isododecane, and by adding additional compounds shown in Table 1 below in the amount shown in Table 1.

Polymer compound H7 and polymer compound G1, which have the polymethylsiloxane main chain, exhibited good solubility in isododecane which was used as a solvent.

The cosmetic material according to Example 3 was evaluated for the feeling of use in the same manner as in Example 1. Table 2 shows the results.

<<Comparative Example 1 (Comp.Ex.1)>>

The cosmetic material according to Comparative Example 1 was produced in the same manner as in Example 1, except that trimethylsiloxysilicate (SR1000, manufactured by MOMENTIVE) was used instead of the isododecane solution of polymer compound H5 and polymer compound G1.

Evaluations of the feeling of use were performed for the cosmetic material according to Comparative Example 1 in the same manner as in Example 1. Table 2 shows the results.

<<Comparative Example 2 (Comp.Ex.2)>>

The cosmetic material according to Comparative Example 2 was produced in the same manner as in Example 1, except that a silicone resin (siloxane cross-linked product, SilForm Flexible Resin (trade name) (manufactured by MOMENTIVE)) was used instead of the isododecane solution of polymer compound H5 and polymer compound G1.

Evaluations of the feeling of use were performed for the cosmetic according to Comparative Example 2 in the same manner as in Example 1. Table 2 shows the results.

[Table 1]

Table 1

| **Components** | Ex.1 | Ex.2 | Ex.3 | Comp.Ex.1 | Comp.Ex.2 |
|---|---|---|---|---|---|
| | pbw | pbw | pbw | pbw | pbw |
| Isododecane solution of polymer compound H5 and polymer compound G1 | 5 | - | - | - | - |
| Isododecane solution of polymer compound H5 and polymer compound G2 | - | 5 | - | - | - |
| Isododecane solution of polymer compound H7 and polymer compound G1 | - | - | 5 | - | - |
| Trimethylsiloxysilicate | - | - | - | 1 | - |
| Silicone resin (SilForm Flexible Resin) | - | - | - | - | 1 |
| Decamethylpentasiloxane | 25 | 25 | 25 | 29 | 29 |
| PPG-17 | 1 | 1 | 1 | 1 | 1 |
| PEG-9 Polydimethylsiloxyethyl Dimeticone | 1.5 | 1.5 | 1.5 | 1.5 | 1.5 |
| Ethylhexyl Methoxycinnamate | 10 | 10 | 10 | 10 | 10 |
| Octocrylene | 3 | 3 | 3 | 3 | 3 |

(continued)

| Components | Ex.1 | Ex.2 | Ex.3 | Comp.Ex.1 | Comp.Ex.2 |
|---|---|---|---|---|---|
| | pbw | pbw | pbw | pbw | pbw |
| Tocopherol | q.s | q.s | q.s | q.s | q.s |
| Isopropyl Myristate | 5 | 5 | 5 | 5 | 5 |
| Bis-Ethylhexyloxyphenol Methoxyphenyl Triazine | 1 | 1 | 1 | 1 | 1 |
| Diethylamino Hydroxybenzoyl Hexyl Benzoate | 2 | 2 | 2 | 2 | 2 |
| Hydrophobized titanium oxide | 2 | 2 | 2 | 2 | 2 |
| Hydrophobized zinc oxide | 10 | 10 | 10 | 10 | 10 |
| Silica | 2 | 2 | 2 | 2 | 2 |
| Talc | 7 | 7 | 7 | 7 | 7 |
| Methyl Methacrylate Crosspolymer | 5 | 5 | 5 | 5 | 5 |
| Water | Balance | Balance | Balance | Balance | Balance |
| Total | 90 | 90 | 90 | 90 | 90 |
| q.s = quantum sufficit<br>pbw = parts by weight | | | | | |

[Table 2]

Table 2

| | Polymer compound having host group | | | Polymer compound having guest group | *a | *b | *c | *d |
|---|---|---|---|---|---|---|---|---|
| | ID | Ester group bound to Si atom | Pentyl group bound to Si atom | ID | | | | |
| Ex.1 | H5 | yes | - | G1 | Good | Good | Good | Moderate |
| Ex.2 | H5 | yes | - | G2 | Good | Good | Good | Moderate |
| Ex.3 | H7 | - | yes | G1 | Good | Very good | Good | Good |
| Comp.Ex. 1 | - | | | - | Poor | Poor | Good | Poor |
| Comp.Ex. 2 | - | | | Silicone resin | Moderate | Poor | Good | Poor |
| (*a) tightness<br>(*b) close adhesiveness<br>(*c) resilience<br>(*d) durability | | | | | | | | |

<<Example 4 (Ex.4)>>

Using polymer compound CH-4 having a host group according to Production Example 15 described above and the same siloxane cross-linked product as used in Comparative Example 2 described above, production and evaluation of a cosmetic material were performed in the same manner as in Example 1. The results are shown in Table 3 below.

<<Example 5 (Ex.5)>>

Using polymer compound CH-4 having a host group according to Production Example 15 described above and polymer compound G2 having a guest group according to Production Example 13 described above, production and evaluation of a cosmetic material were performed in the same manner as in Example 1. The results are shown in Table 3 below.

[Table 3]

Table 3

| | Polymer compound having host group | | Polymer compound having guest group | | *a | *b | *c | *d |
|---|---|---|---|---|---|---|---|---|
| | ID | Siloxane bond | ID | Siloxane bond | | | | |
| Ex.4 | CH-4 | no | Silicone resin | yes | Good | Good | Good | Moderate |
| Ex.5 | CH-4 | no | G2 | yes | Moderate | Good | Good | Moderate |
| Comp.Ex. 1 | | - | - | | Poor | Poor | Good | Poor |
| Comp.Ex. 2 | | - | Silicone resin | yes | Moderate | Poor | Good | Poor |

(*a) tightness
(*b) close adhesiveness
(*c) resilience
(*d) durability

**Claims**

1. A cosmetic material comprising a host body consisting of a polymer compound having a host group and a guest body consisting of a polymer compound having a guest group, or a host-guest body consisting of a polymer compound having a host group and a guest group, wherein, at least one of the polymer compound having the host group and the polymer compound having the guest group, or the polymer compound having the host group and the guest group, has a siloxane bond.

2. The cosmetic material according to claim 1, wherein the polymer compound having the host group and the polymer compound having the guest group have a siloxane bond.

3. The cosmetic material according to claim 1, wherein at least one of the polymer compound having the host group and the polymer compound having the guest group, or the polymer compound having the host group and the guest group, has a siloxane bond in the main chain of the polymer.

4. The cosmetic material according to claim 3, wherein the polymer compound having the host group and the polymer compound having the guest group have a siloxane bond in the main chain of the polymer.

5. The cosmetic material according to one of claims 1 to 4, wherein

   the polymer compound having the host group has the host group in a side chain thereof, and
   the polymer compound having the guest group has the guest group in a side chain thereof.

6. The cosmetic material according to one of claims 1 to 4, wherein

the polymer compound having the host group has the host group in a side chain thereof, and
the polymer compound having the guest group has the guest group in a terminal thereof.

**7.** The cosmetic material according to one of claims 1 to 4, wherein

the polymer compound having the host group has the host group in a terminal thereof, and
the polymer compound having the guest group has the guest group in a side chain thereof.

**8.** The cosmetic material according to one of claims 1 to 7, wherein at least one of the polymer compound having the host group and the polymer compound having the guest group, or the polymer compound having the host group and the guest group, is cross-linked.

**9.** The cosmetic material according to one of claims 1 to 8, wherein at least one of the polymer compound having the host group and the polymer compound having the guest group, or the polymer compound having the host group and the guest group, has a structure represented by the following formula (1):

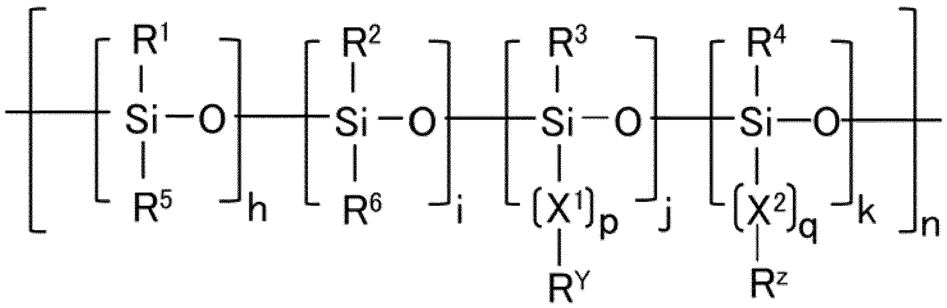

· · · (1)

[In Formula (1), $R^1$ to $R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, a monovalent spirocyclic compound, a monovalent condensed-ring compound, a group represented by $-R^{R1}$ -COOH, or a group represented by $-R^{R2}$-C(O)O-$R^{R3}$ , wherein these groups may have a substituent, and wherein $R^{R1}$, $R^{R2}$ and $R^{R3}$ are each an alkyl group or alkylene group having 1 to 10 carbon atoms; $X^1$ and $X^2$ are each independently O, $Si(OH)_2$, $Si(R^{10})_2$, N(H) or $N(COCH_3)$, or an urethane bond, an urea bond, an ether bond, an amide bond, or an ester bond, or a carbonyl group, an alkylene group, a cycloalkylene group, an alkenylene group, an alkoxylene group, a divalent heterocyclic group, an urethane group, an urea group, or an arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j, and k each represent an integer of 0 or more; and they may be the same or different from each other; at least j or k represents an integer of 1 or more; n represents an integer of 1 or more.]

**10.** The cosmetic material according to claim 9, wherein the polymer compound having the host group and the polymer compound having the guest group have the structure represented by the formula (1).

**11.** The cosmetic material according to claim 9 or 10, wherein at least one of the polymer compound having the host group and the polymer compound having the guest group, or the polymer compound having the host group and the guest group, has the structure represented by the formula (1) in the main chain of the polymer.

**12.** The cosmetic material according to claim 9 or 10, wherein at least one of the polymer compound having the host group and the polymer compound having the guest group, or the polymer compound having the host group and the guest group, has:

a vinyl main chain, an acryl main chain, an urethane main chain, an epoxy main chain, a polyimide main chain, a polyester main chain, a poly-urea main chain, or a polycarbonate main chain, and
a side chain having the structure represented by the formula (1).

**13.** The cosmetic material according to one of claims 1 to 12, wherein at least one of the polymer compound having the host group and the polymer compound having the guest group, or the polymer compound having the host group and the guest group, has a structural unit represented by the following formula (2):

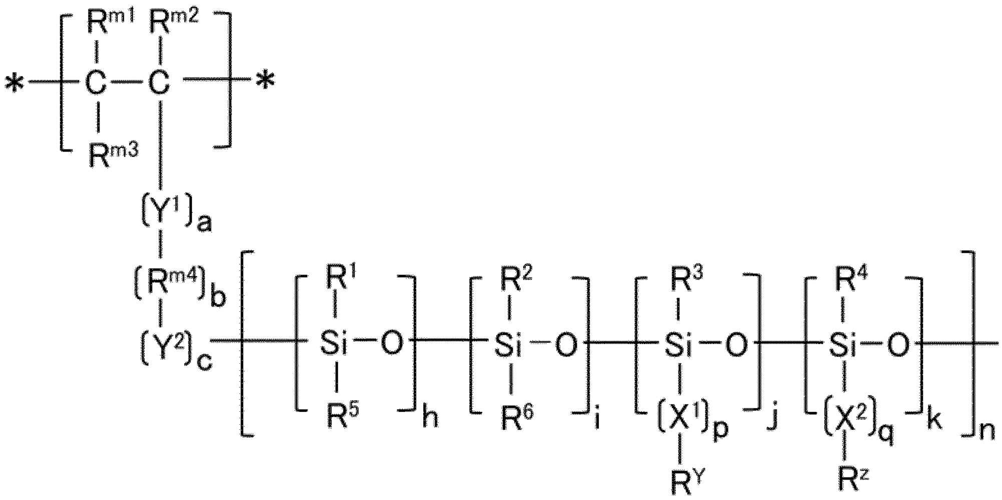

$\cdots$ (2)

[In Formula (2), $R^1$-$R^6$ are each independently a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, a monovalent heterocyclic group, a monovalent spirocyclic compound, a monovalent condensed-ring compound, a group represented by -$R^{R1}$ -COOH, or a group represented by -$R^{R2}$-C(O)O-$R^{R3}$, wherein these groups may have a substituent, and wherein $R^{R1}$, $R^{R2}$ and $R^{R3}$ are each an alkyl group or alkylene group having 1 to 10 carbon atoms; $X^1$ and $X^2$ are each independently O, $Si(OH)_2$, $Si(R^{10})_2$, N(H), or $N(COCH_3)$, or an urethane bond, an urea bond, an ether bond, an amide bond, or an ester bond, or a carbonyl group, an alkylene group, a cycloalkylene group, an alkenylene group, an alkoxylene group, a divalent heterocyclic group, an urethane group, an urea group, or an arylene groups, and these groups may have a substituent; $R^{10}$ is a hydrogen atom, or an alkyl group, a cycloalkyl group, an alkenyl group, an alkoxy group, a cycloalkoxy group, a hydroxy group, a carboxyl group, an aldehyde group, an aryl group, an aryloxy group, or a heterocyclic group, and these groups may have a substituent; a plurality of $R^{10}$ may be the same or different; each of $X^1$ and $X^2$ may be the same or different if there are a plurality of them; p and q each independently represent an integer of 0 or more; $R^Y$ is a host group; $R^Z$ is a guest group; h, i, j, and k each represent an integer of 0 or more, these may be the same or different from each other, and at least j or k is an integer of 1 or more; n represents an integer of 1 or more; $R^{m1}$ to $R^{m3}$ are each independently a hydrogen atom or an alkyl group, $Y^1$ is an ether bond, an amide bond, or an ester bond; $R^{m4}$ is an alkylene group, a cycloalkylene group, an alkenylene group, an alkoxylene group, or an arylene group, and these groups may have a substituent, and $Y^2$ is an ether bond, an amide bond, or an ester bond, O, Si $(OH)_2$, $Si(R^{10})_2$,N(H), or $N(COCH_3)$; and a, b, and c each independently represent an integer of 0 to 3; * represents a single bond constituting the main chain of the polymer compound.]

**14.** The cosmetic material according to claim 13, wherein the polymer compound having the host group and the polymer compound having the guest group have the structure represented by the formula (2).

**15.** The cosmetic material according to claim 13 or 14, wherein at least one of the polymer compound having the host group and the polymer compound having the guest group, or the polymer compound having the host group and the guest group, further has a structural unit represented by the following formula (3):

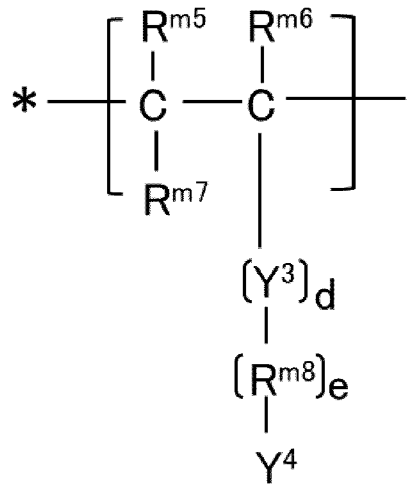

[In Formula (3), $R^{m5}$ to $R^{m7}$ are each independently a hydrogen atom or an alkyl group; $Y^3$ is an ether bond, an amide bond, or an ester bond; $R^{m8}$ is an alkylene group, a cycloalkylene group, an alkenylene bond, an alkoxylene group, or an arylene group, and these groups may have a substituent; $Y^4$ is a hydrogen atom, or an alkyl group, a hydroxyl group, $Si(OH)_3$, $Si(R^{10})_3$, $NH_2$, $C(O)CH_3$, $C(O)NH_2$, or $N(COCH_3)$; d and e are each independently an integer of 0 to 3; * represents a single bond constituting the main chain of the polymer compound.]

**16.** The cosmetic material according to one of claims 9 to 15, wherein, in the formula (1) and/or formula (2),

the host group represented by $R^Y$ is $\alpha$-cyclodextrin, $\beta$- cyclodextrin or $\gamma$- cyclodextrin, and
the guest group represented by $R^Z$ is an alkyl group which can have a substituent, or an aryl group which can have a substituent.

**17.** The cosmetic material according to one of claims 9 to 16, wherein, in the formula (1) and/or formula (2), $R^1$, $R^2$, $R^3$, $R^4$ and $R^5$ are a methyl group.

**18.** The cosmetic material according to one of claims 9 to 16, wherein, in the formula (1) and/or formula (2),

k=0, and
$R^1$, $R^2$, $R^3$ , $R^5$ and $R^6$ are an alkyl group.

**19.** The cosmetic material according to one of claims 9 to 16, wherein, in the formula (1) and/or formula (2),

k=0
$R^1$, $R^2$, $R^3$ and $R^5$ are a methyl group, and
$R^6$ is a pentyl group.

**20.** The cosmetic material according to one of claims 9 to 19, wherein, in the formula (1) and/or formula (2),

$(X^1)_p$ is $-(CH_2)_3-N(COCH_3)-$ and j is an integer of 1 or more,
and/or
$(X^2)_q$ is $-R^{11}-CO-O-$ or $-R^{11}-CO-NH-$, wherein $R^{11}$ is an alkylene group having 1 to 12 carbon atoms, which can have a substituent, and k is an integer of 1 or more.

**21.** The cosmetic material according to one of claims 1 to 20, wherein the host group and the guest group are selected from one of the following combinations (a) to (c):

(a) the host group is $\alpha$-cyclodextrin and
the guest group is at least one selected from the group consisting of (1) a linear alkyl group of 4 to 18 carbon atoms, (2) a linear alkyl group of 4 to 18 carbon atoms having a hydroxyl group, (3) a linear alkyl group of 4 to 18 carbon atoms having a carboxyl group, (4) a linear alkyl group of 4 to 18 carbon atoms having an amino group, (5) a cyclic alkyl group, (6) a phenyl group, (7) an azobenzene group, and (8) a cinnamic acid group;

(b) the host group is β-cyclodextrin and
the guest group is at least one selected from the group consisting of (1') t-butyl group, (2') an adamantyl group, (3') an aryl group, (4') an aryl group having a hydroxyl group, (5') an aryl group having a carboxyl group, (6') an aryl group having an amino group, (7') a ferrocenyl group, (8') an azobenzene group, and (9') a dansyl group;
(c) the host group is γ-cyclodextrin and

the guest group is at least one selected from the group consisting of (1") an alkyl group of up to 18 carbon atoms, (2") an alkyl group of up to 18 carbon atoms having a hydroxy group, (3") an alkyl group of up to 18 carbon atoms having a carboxyl group, (4") an alkyl group of up to 18 carbon atoms having an amino group, (5") an adamantyl group, (6") a group having clusters composed of carbon atoms, (7") a dansyl group having an aryl group, (8") a ferrocenyl group, and (9") an anthracenyl group.

**22.** The cosmetic material according to one of claims 1 to 19, further comprising a solvent.

**23.** The cosmetic material according to claim 20, wherein the solvent is volatile silicone or a volatile hydrocarbon compound.

## INTERNATIONAL SEARCH REPORT

International application No.
PCT/JP2021/007182

A. CLASSIFICATION OF SUBJECT MATTER
A61K 8/89(2006.01)i; A61Q 19/00(2006.01)i
FI: A61K8/89; A61Q19/00
According to International Patent Classification (IPC) or to both national classification and IPC

B. FIELDS SEARCHED
Minimum documentation searched (classification system followed by classification symbols)
A61K8/89; A61Q19/00

Documentation searched other than minimum documentation to the extent that such documents are included in the fields searched
Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| X<br>A | JP 2005-536653 A (WACKER-CHEMIE GMBH) 02 December 2005 (2005-12-02) claims, paragraphs [0030]-[0094], [0212]-[0214] | 1, 3, 9, 11, 16-17, 20-23<br>2, 4-8, 10, 12-15, 18-19 |
| A | JP 2015-512955 A (MOMENTIVE PERFORMANCE MATERIALS INC.) 30 April 2015 (2015-04-30) entire text | 1-23 |
| A | US 2018/0362814 A1 (CITY UNIVERSITY OF HONG KONG) 20 December 2018 (2018-12-20) entire text | 1-23 |
| A | CN 110746943 A (UNIV CHINA PETROLEUM EAST CHINA) 04 February 2020 (2020-02-04) entire text | 1-23 |
| E, A | WO 2021/045203 A1 (OSAKA UNIVERSITY) 11 March 2021 (2021-03-11) entire text | 1-23 |

☐ Further documents are listed in the continuation of Box C.
☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 April 2021 (08.04.2021) | 27 April 2021 (27.04.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2021/007182

| Patent Documents referred in the Report | Publication Date | Patent Family | Publication Date |
|---|---|---|---|
| JP 2005-536653 A | 02 Dec. 2005 | US 2006/0009592 A1<br>claims, paragraphs [0044], [0185]-[0187]<br>WO 2004/018547 A1<br>EP 1865016 A1<br>DE 10238818 A1<br>CN 1678665 A<br>AU 2003255443 A1 | |
| JP 2015-512956 A | 30 Apr. 2015 | US 2013/0172419 A1<br>entire text<br>WO 2013/103536 A1<br>EP 2800777 A1<br>CN 104136500 A<br>KR 10-2014-0116905 A | |
| US 2018/0362814 A1 | 20 Dec. 2018 | (Family: none) | |
| CN 110746943 A | 04 Feb. 2020 | (Family: none) | |
| WO 2021/045203 A1 | 11 Mar. 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 201999576 A
- WO 2012036069 A
- WO 2013162019 A


### Non-patent literature cited in the description


- *Tetrahedron Letters,* 1984, vol. 25 (31), 3331-3334
- *Macromolecules,* 2019, vol. 52 (7), 2659-2668